(19)

**Europäisches Patentamt**

**European Patent Office**

**Office européen des brevets**

(11) **EP 3 342 465 A1**

(12) **EUROPEAN PATENT APPLICATION**

(43) Date of publication:
04.07.2018 Bulletin 2018/27

(21) Application number: 16207612.9

(22) Date of filing: 30.12.2016

(51) Int Cl.:
*A61Q 17/00* (2006.01)          *A61Q 17/04* (2006.01)
*A61K 8/97* (2017.01)           *A61Q 19/02* (2006.01)
*A61Q 19/08* (2006.01)

(84) Designated Contracting States:
AL AT BE BG CH CY CZ DE DK EE ES FI FR GB GR HR HU IE IS IT LI LT LU LV MC MK MT NL NO PL PT RO RS SE SI SK SM TR
Designated Extension States:
BA ME
Designated Validation States:
MA MD

(71) Applicant: **Bayer Consumer Care AG**
**4052 Basel (CH)**

(72) Inventor: **The designation of the inventor has not yet been filed**

(74) Representative: **BIP Patents**
**c/o Bayer Intellectual Property GmbH**
**Alfred-Nobel-Straße 10**
**40789 Monheim am Rhein (DE)**

(54) **HEDYCHIUM EXTRACT AND COMPOSITIONS THEREOF AND THEIR USE IN THE TREATMENT OF SKIN AFFECTED BY HARMFUL ENVIRONMENTAL INFLUENCES**

(57)    The present invention relates to the use of the root of the plant *Hedychium coronarium J. Koenig* and extracts derived therefrom in cosmetic applications for the treatment of skin affected by harmful environmental influences such as pollutants and UV radiation. The invention further relates to cosmetic compositions comprising *Hedychium coronarium J. Koenig* root extract and a process for preparing *Hedychium coronarium J. Koenig* root extract.

EP 3 342 465 A1

**Description**

**FIELD OF THE INVENTION**

The present invention relates to the use of the plant *Hedychium* and extracts derived therefrom in cosmetic applications for the treatment of skin affected by harmful environmental influences. The invention further relates to cosmetic compositions comprising *Hedychium* extract and a process for preparing *Hedychium* extract.

**BACKGROUND OF THE INVENTION AND PRIOR ART**

*Hedychium* is a genus of flowering plants in the ginger family *Zingiberaceae.* There are approximately 70-80 known species, native to Southeast Asia (Thailand, Malaysia, Indonesia, the Philippines, etc.), southern China, the Himalayas and Madagascar. Some species have become widely naturalized in other lands (South Africa, South America, Central America, the West Indies, and many of the islands of the Pacific, Indian and Atlantic Oceans). The genus name *Hedychium* is derived from two ancient Greek words, *hedys* meaning "sweet" and *chios* meaning "snow". This refers to the fragrant white flower of the type species *Hedychim coronarium.* Common names include garland flower, ginger lily, and kahili ginger.

Members of the genus *Hedychium* are rhizomatous perennials, commonly growing 120-180 cm (47-71 in) tall. Some species are cultivated for their exotic foliage and fragrant spikes of flowers in shades of white, yellow and orange. Numerous cultivars have been developed for garden use. Selected species comprise for example *Hedychium aurantiacum, Hedychium coccineum, Hedychium coronarium, Hedychium densiflorum, Hedychium ellipticum* (shaving brush ginger), *Hedychium flavescens, Hedychium gardnerianum* (ginger lily), *Hedychium samuiense*, and *Hedychium spicatum* called *kapur kachari* in Hindi).

The individual species differ from each other in their individual biological taxonomy and is several cases also with respect to their origin.

For example, the species *Hedychium coronarium* (also known as "ginger butterfly") was described in 1783 by Johann Gerhard Koenig in the book of Andrea Johan Retzius "Observationes Botanicae" t. 3 pages 73-74 and has accordingly been named by said author as *Hedychium coronarium* J. Koenig, also known under the common names butterfly-ginger; cinnamon-jasmine, garland-flower; gingy lily; white ginger, jiang hua, sweet-scented garland flower, gingembre sauvage, longose blanc, gingembre papillon. The origin of *Hedychium coronarium* J. Koenig is Madagascar, South Asia, India and Malaysia.

In contrast, the species *Hedychium spicatum* (also known as "wild-headed ginger") was first recorded by James Edward Smith (1811), and then described by Francis Buchanan-Hamilton in 1819 in the work of Abraham REES "the Cyclopaedia; universal dictionnary Arts, Sciences and Littérature." t. 17 p.521-522 and has accordingly been named by said authors as *Hedychium spicatum* Buch.-Ham. ex Sm, also known under the common names long-spiked garland-flower, cao guo yao, gingembre sauvage à épis and many more common names exist in India and Nepal. The origin of *Hedychium spicatum* Buch.-Ham. ex Sm is the Himalaya, Nepal, the North-East of India, North of Thailand, and China (Guizhou, Sichua, Xizang, Yunnan).

*Hedychium* has widely been described for use in traditional medicine as described for example in "Edible Medicinal and non medicinal Plants", Vol. 8 Flowers, pages 853-860 or in "Medicinal plants used by women from Agnalazaha littoral forest (Sotuhestern Madagascar)", Journal of Ethnobiology and Ethnomedicine, 2013 as well as by X. Yan, et al. "Traditional Chinese Medicines, Molecular Structures, Natural Sources, and Applications", 1999 or by Sharma et al. "Phytochemistry", 14:578, 1975.

Some *Hedychium* species have also been described for cosmetic use. For example WO 2002/056859 A2 relates to compositions comprising *Hedychium* extract and the cosmetic use thereof. However, said international application particularly describes the use of the species *Hedychium spicatum* and in one aspect its activity in regulating the firmness, tone, or texture of skin and in another aspect its use in the treatment of environmental damage in skin based on the underlying mechanisms of inhibiting UV induced metalloproteinase-1 (MMP-1) secretion, preventing smoke-induced loss of thiols to protect the glutathione as a part of the endogenous cellular antioxidant defense system, and inhibiting nitric oxide production as a precursor for the formation of harmful reactive oxygen species (ROS). Accordingly, WO 2002/056859 A2 describes the use of the *Hedychium spicatum* species for the cosmetic use of regulating firmness, tone, or texture of skin as well as of treating UV induced damages to skin by influencing the antioxidant and ROS defense system.

BRPI0905586-0 A2 describes the cosmetic use of *Hedychium coronarium* extracts derived from the flowers of the plant for moisturizing, revitalizing, and regenerating the skin. Further, an anti-aging effect due to high concentration of flavonoids in the flowers is mentioned as well as an anti-inflammatory and anti-peroxidant activity, and a strengthening of micro vessels and capillaries, and the property of combating the formation of edemas and photo-induced erythema. The use or any cosmetic efficiency of extracts derived from the roots of *Hedychium coronarium* is not described therein

Also WO 2006/053415 A1 mentions the suitability of several plant extracts in cosmetic applications, including applications making use of the mechanism of inhibition of MMP-1, MMP-2, MMP-3, MMP-9, HLE (human leukocyte elastase). However, therein *Hedychium* plant extracts are only mentioned very generally in a large list of possible plants and no specific effect has been ascribed to or shown for any *Hedychium* species.

## OBJECT TO BE SOLVED

It was the object of the present invention to provide a new cosmetically active agent being suitable for the cosmetic use in the treatment of skin affected by harmful environmental influences.

The problem has been solved by the inventors of the present invention, who surprisingly found that *Hedychium* and extracts derived therefrom exhibit activity in several biophysiological mechanisms on a cellular basis and are thus actively influencing the cellular clean-up system, thus helping to protect cells against harmful environmental impacts and to treat cellular and skin damages deriving therefrom.

Surprisingly, the inventors of the present invention found that in particular the specific species *Hedychium coronarium* J. Koenig exhibits the new found activity in protecting and/or activating at least one of the cellular clean-up system, thus being particularly suitable in the protection against and/or the treatment of environmental damages.

Very particularly, the inventors of the present invention found that in particular extracts derived from the roots (rhizomes) of the specific species *Hedychium coronarium* J. Koenig exhibits the new found activity in protecting and/or activating at least one of the cellular clean-up system, thus being particularly suitable in the protection against and/or the treatment of environmental damages.

## DETAILED DESCRIPTION OF THE INVENTION

The present invention generally relates to the use of plants of the *Hedychium* family or of an extract thereof as a cosmetic agent in the treatment of skin affected by harmful environmental influences. More particularly, the invention relates to the use of plants of the *Hedychium* family or of an extract thereof as a cosmetic agent in the protection against and/or the treatment of environmental damages of the skin.

Unless defined otherwise, all technical and scientific terms used herein have the same meaning as commonly understood by one of ordinary skill in the art to which the invention belongs.

In the sense of the present invention plants of the *Hedychium* family generally comprise all plants belonging to said botanical plant family as known by a person skilled in the art. The plants of the *Hedychium* family comprise for example the species *Hedychium aurantiacum, Hedychium coccineum, Hedychium coronarium, Hedychium densiflorum, Hedychium ellipticum* (shaving brush ginger), *Hedychium flavescens, Hedychium gardnerianum* (ginger lily), *Hedychium samuiense*, and *Hedychium spicatum* called *kapur kachari* in Hindi). According to the invention the species *Hedychium coronarium* and *Hedychium spicatum* are preferred. Most preferred is the species *Hedychium coronarium,* such as very particularly the species *Hedychium coronarium* J. Koenig, which has been described in detail above.

According to the invention it is particularly preferred to use an extract of the *Hedychium* plant or any part thereof as the cosmetically active agent. In principle a plant extract can be derived from flowers, seeds, fruits, leaves, stem or roots (and rhizomes) of the plants. It is particularly preferred to use an extract derived from the roots. Therein, the term "roots" comprises roots as well as rhizomes of the plant and the terms may also be used interchangeably.

Accordingly, the present invention particularly relates to the cosmetic use as defined herein of a *Hedychium* root and/or rhizome extract, more particularly to a *Hedychium coronarium J. Koenig* root and/or rhizome extract, such as to the cosmetic use in the protection against harmful environmental influences and/or in the protection against and/or the treatment of environmental damages deriving therefrom.

In the context of the present invention the term "extract" means an agent derived by extraction of similar or different parts of the *Hedychium* plant, wherein extraction is a generally known process for a person skilled in the art. An extract in accordance with the present invention is a blend of compounds isolated from the extracted part(s) of the plant from the Hedychium genus, such as in particular form the *Hedychium coronarium* J. Koenig species. In principle, such compounds may be isolated from any part(s) of the plant as mentioned above, by using extraction procedures well known in the art, such as in particular by using one or more organic solvents.

Examples of suitable solvents include lower $C_1$-$C_8$-alcohols $C_1$-$C_8$-alkyl polyols, $C_1$-$C_8$-alkyl ketones, $C_1$-$C_8$-alkyl ethers, acetic acid, $C_1$-$C_8$-alkyl esters, chloroform, and/or inorganic solvents such as water, inorganic acids such as hydrochloric acid, and inorganic bases such as sodium hydroxide, and mixtures thereof. Preferred solvents are hydrophilic (water and water miscible) solvents. Preferred solvents include ethanol, 1-propanol, iso-propyl alcohol, ethyl acetate, butyl acetate, glycerol, propylene glycol, liquid polyethylene glycols etc. and mixtures thereof. Most preferred are ethanol, water and glycerol and mixtures thereof.

Suitable mixtures of a solvent for the *Hedychium* extract may comprise water and glycerol in a ratio of 10 : 90 to 90 : 10, preferably 15 : 85 to 85 : 15, preferably 20 : 80 to 80 : 20, preferably 25 : 75 to 75 : 25, preferably 30 : 70 to 70 : 30, or 35 : 65 to 65 :35, or 40 : 60 to 60 : 40, or 45 : 55 to 55 : 45, or 50 : 50.

According to the present invention a *Hedychium* extract, as defined herein, having a concentration of $\leq 10.0$ % (weight / volume), $\leq 9.0$ % (weight / volume), $\leq 8.0$ % (weight / volume), $\leq 7.0$ % (weight / volume), $\leq 6.0$ % (weight / volume), $\leq 5.5$ % (weight / volume), $\leq 5.0$ % (weight / volume), $\leq 4.5$ % (weight / volume), $\leq 4.0$ % (weight / volume), ), $\leq 3.5$ % (weight / volume), ), $\leq 3.4$ % (weight / volume), $\leq 3.3$ % (weight / volume), $\leq 3.2$ % (weight / volume), ), $\leq 3.1$ % (weight / volume), $\leq 3.0$ % (weight / volume), $\leq 2.9$ % (weight / volume), ), $\leq 2.8$ % (weight / volume), $\leq 2.7$ % (weight / volume), $\leq 2.6$ % (weight / volume), ), $\leq 2.5$ % (weight / volume), $\leq 2.4$ % (weight / volume), ), $\leq 2.3$ % (weight / volume), $\leq 2.2$ % (weight / volume), $\leq 2.1$ % (weight / volume), ), $\leq 2.0$ % (weight / volume), or $\leq 1.9$ % (weight / volume), ), :5 1.8 % (weight / volume), $\leq 1.7$% (weight / volume), $\leq 1.6$% (weight / volume), ), $\leq 1.5$ % (weight / volume), $\leq 1.4$ % (weight / volume), ), $\leq 1.3$ % (weight / volume), $\leq 1.2$ % (weight / volume), $\leq 1.1$% (weight / volume), ), $\leq 1.0$ % (weight / volume), or < 1.0 % (weight / volume), such as $\leq 0.5$ % (weight / volume) may be used, based on the composition of the extract.

Preferably, the *Hedychium* extract, as defined herein, has a concentration of $\geq 0.0005$ % (weight / volume), preferably $\geq 0.001$ % (weight / volume), $\geq 0.002$ % (weight / volume), $\geq 0.003$ % (weight / volume), $\geq 0.004$ % (weight / volume), $\geq 0.005$ % (weight / volume), $\geq 0.006$ % (weight / volume), $\geq 0.007$ % (weight / volume), $\geq 0.008$ % (weight / volume), $\geq 0.009$ % (weight / volume), $\geq 0.01$ % (weight / volume), $\geq 0.02$ % (weight / volume), $\geq 0.03$ % (weight / volume), $\geq 0.04$ % (weight / volume), $\geq 0.05$ % (weight / volume), $\geq 0.06$ % (weight / volume), $\geq 0.07$ % (weight / volume), $\geq 0.08$ % (weight / volume), $\geq 0.09$ % (weight / volume), $\geq 0.1$ % (weight / volume), $\geq 0.2$ % (weight / volume), $\geq 0.3$ % (weight / volume), $\geq 0.4$ % (weight / volume), $\geq 0.5$ % (weight / volume).

Preferably, the *Hedychium* extract, as defined herein, has a concentration in the range of 0.01 to 10.0 % (weight / volume), $\leq 9.0$ % (weight / volume), $\leq 8.0$ % (weight / volume), $\leq 7.0$ % (weight / volume), $\leq 6.0$ % (weight / volume), $\leq 5.5$ % (weight / volume), $\leq 5.0$ % (weight / volume), $\leq 4.5$ % (weight / volume), $\leq 4.0$ % (weight / volume), ), $\leq 3.5$ % (weight / volume), ), $\leq 3.4$ % (weight / volume), $\leq 3.3$ % (weight / volume), $\leq 3.2$ % (weight / volume), ), $\leq 3.1$ % (weight / volume), $\leq 3.0$ % (weight / volume), $\leq 2.9$ % (weight / volume), ), $\leq 2.8$ % (weight / volume), $\leq 2.7$ % (weight / volume), $\leq 2.6$ % (weight / volume), ), :5 2.5 % (weight / volume 2.4 % (weight / volume), ), $\leq 2.3$ % (weight / volume), $\leq 2.2$ % (weight / volume), $\leq 2.1$ % (weight / volume), ), $\leq 2.0$ % (weight / volume), or $\leq 1.9$ % (weight / volume), ), $\leq 1.8$ % (weight / volume), $\leq 1.7$ % (weight / volume), $\leq 1.6$ % (weight / volume), ), $\leq 1.5$ % (weight / volume), $\leq 1.4$ % (weight / volume), ), $\leq 1.3$ % (weight / volume), $\leq 1.2$ % (weight / volume), $\leq 1.1$ % (weight / volume), ), $\leq 1.0$ % (weight / volume), or < 1.0 % (weight / volume), such as $\leq 0.5$ % (weight / volume) may be used, based on the composition of the extract.

The *Hedychium* extract, as defined herein, may also have a concentration in a range with any upper limit selected from the "$\leq$" values as defined above and any lower limit selected from the "$\geq$" values as defined above. Preferred is a range of 0.05 to 5.0 % (weight / volume), more preferred of 0.1 to 3.0 % (weight / volume), even more preferred of 0.2 to 2.0 % (weight / volume).

According to the present invention the *Hedychium* plant or the extract thereof is particularly suitable as a cosmetic agent for the cosmetic use in the treatment of skin affected by harmful environmental influences, such as in particular for the protection against and/or the treatment of environmental damages of the skin. Therein, harmful environmental influences comprise, without being limited thereto, harmful influences from toxins and/or pollutants, including exhaust, industrial pollution, agricultural pollution, and cigarette smoke, as well as harmful radiation, such as UV radiation, e.g., from the sun or non-natural sources including UV lamps and solar simulators, and ozone.

According to the present invention environmental damages comprise deterioration or damages to the natural skin metabolism and/or natural skin barrier system, which directly or indirectly derive from the aforesaid harmful environmental influences, including for example UV damages, oxidative and/or nitrosative damage to and modifications on lipids, carbohydrates, peptides, proteins, nucleic acids, and vitamins. Effects of environmental damage on the skin also include, without being limited thereto, loss of cell viability, loss or alteration of cell functions, and changes in gene and/or protein expression. In particular, environmental damage according to the present invention includes intracellular damages, comprising damages in mitochondria, lysosomes, and in the cellular transmembrane system, but also damages effecting inflammatory processes and oxidative processes, such as.

As used herein, "protection against environmental damage" means the prevention and/or reduction of symptoms or of the harmful effects deriving from the harmful environmental influences.

As used herein, "treatment of environmental damage" means the reduction, amelioration, improvement and/or elimination of symptoms or damages deriving from the harmful environmental influences.

The inventors of the present invention have surprisingly found, that *Hedychium* or an extract thereof, as defined herein, is particularly suitable in the cosmetic use for the protection against harmful environmental influences and the protection against and/or treatment of environmental damages deriving therefrom by protecting or activating at least one of the cellular clean-up system.

The cellular clean-up system according to the present invention comprises cellular systems of the cell metabolism (hereinafter also referred to as "cellular metabolic system") and of the intracellular barrier system, which are essential for providing viable and intact cells.

Therein, cellular systems of the cellular metabolic system and of the intracellular barrier system, comprise in particular the following systems:

The mitochondria generate most of the cell's supply of adenosine triphosphate (ATP), used as a source of chemical energy and thus represent the so-called power supply of the cells. In addition to supplying cellular energy, mitochondria are involved in a range of other crucial cell processes, such as signaling, thermogenesis, cellular differentiation, control of the cell division cycle and cell growth, cell death (apoptosis), oxidative metabolism, homeostasis of glucids, lipids, calcium, iron (heme synthesis), and much more. Therefrom it becomes apparent that viable and vital mitochondria are a prerequisite for a viable and vital cell and a functioning cellular defense system against harmful environmental influences.

The lysosomes are cytoplasmic organelles that permit recycling of cellular materials that have exceeded their lifetime or are otherwise no longer useful. Their main function is the digestion of endogenous or exogenous substrates (so called autophagy or heterophagy), in all eukaryotic cells. Lysosomes break down cellular waste products, fats, carbohydrates, proteins, and other macromolecules into simple compounds, which are then transferred back into the cytoplasm as new cell-building materials. Indeed, its lipid membrane contains many enzymes (about 40 different types of hydrolytic enzymes), protons pumps and transport proteins. Acid pH is regulated to allow optimum activities of acid hydrolases (so a lysosome is like a cell stomach). Autophagy is an important mechanism that allows the cell to mobilize its energy stocks to defend and destroy its damaged organelles and then avoid serious effects. This process permits elimination and replacement of proteins and non-functional organelles, then to ensure homeostasis. It is involved in longevity control and development of pathologies as cancer or diabetes. Therefrom it becomes apparent that viable and vital lysosomes are a prerequisite for a viable and vital cell and a functioning cellular defense system against harmful environmental influences.

Also the intracellular barrier system, comprising in particular the cellular transmembrane system, plays an important role in the cellular clean-up system of the cells, as the cellular transmembrane system controls and effects the transport of the metabolic energy products from the mitochondria and of the metabolic waste products from the lysosomes and thus plays a key role in an effective and functioning cell metabolism which are essential for providing viable and intact cells. A strong and intact transmembrane system comprises so called "tight junctions", i.e. junctions established between cells (cell-cell junctions).These junctions are made of several families of transmembrane proteins, such as claudins, occludin and adhesion molecules (ZO-1, ZO-2, ZO-3,...). Tight junctions exist in skin tissue, including epidermis layers. Claudins 1 and 4 are proteins found in upper layers of epidermis (where keratinocytes differentiate). Immunostainings studies have shown the presence of these markers and that these proteins participate in paracellular skin barrier by controlling the flow of molecules in the intercellular space between the cells of an epithelium, as well as by blocking the entry of small molecules and by maintaining the integrity (cohesion reinforced between corneocytes) of the epidermis' upper layers. Furthermore, claudins play a role in the homeostasis of the stratum corneum and the control of the calcium gradient. Damage of the tight junctions leads to damage of the skin barrier due to increasing calcium flux and disturbed gradient, leading to an altered differentiation and finally to a damage of the skin barrier protection. Therefrom it becomes apparent that claudins are an important element of a functioning transmembrane system which is a further key element of viable and vital cells and a functioning cellular defense system against harmful environmental influences.

A further important aspect of the cellular metabolic system relates to the production of interleukin 8 (IL-8), a chemokine produced by macrophages and other cell types such as epithelial cells and endothelial cells. Endothelial cells store IL-8 in their storage vesicles, the Weibel-Palade bodies. IL-8 is initially produced as a precursor peptide of 99 amino acids long which then undergoes cleavage to create several active IL-8 isoforms. IL-8 is secreted and is an important mediator of the immune reaction in the innate immune system response. IL-8, also known as neutrophil chemotactic factor, has two primary functions. It induces chemotaxis in target cells, primarily neutrophils but also other granulocytes, causing them to migrate toward the site of infection. IL-8 also induces phagocytosis once they have arrived. IL-8 is also known to be a potent promoter of angiogenesis. In target cells, IL-8 induces a series of physiological responses required for migration and phagocytosis, such as increases in intracellular $Ca^{2+}$, exocytosis (e.g. histamine release), and the respiratory burst. IL-8 can be secreted by any cells with toll-like receptors that are involved in the innate immune response. Usually, it is the macrophages that see an antigen first, and thus are the first cells to release IL-8 to recruit other cells. Both monomer and homodimer forms of IL-8 have been reported to be potent inducers of the chemokine receptors CXCR1 and CXCR2. Therefrom, it becomes apparent, that a functioning cellular metabolic system further requires a functioning IL-8 production, which, due to its importance for the immune reaction and thus defense system of the cells, plays a further important role in the cellular clean-up system of the cells, thus being a further essential aspect for providing viable and vital cells and a functioning cellular defense system against harmful environmental influences, as it efficiently fights inflammatory stress mediators, which may be induced by pollutants and environmental toxins.

A further important aspect of the cellular metabolic system relates to the production of β-endorphins. β-endorphin are neuromediators, endogenous opioids, derived from Proopiomelanocortin (POMC) that are involved in various biological phenomena as skin physiology and homeostasis, neurotrophic activity, pain, immune defense, endocrinal, emotional and stress responses In skin, β-endorphins are secreted by keratinocytes and its receptors are present in main skin cells (keratinocytes, fibroblasts and melanocytes). Therewith, the β-endorphin are said to play an important role in various skin metabolism and skin defense processes, comprising stimulation of keratinocyte migration, involvement in wound healing and cellular differentiation, induction of epidermal and follicular melanogenesis, and control of hair growth. Furthermore, increased β-endorphin modulates the number of dendritic processes of hair follicle melanocytes. Therefrom, it becomes apparent, that a functioning cellular metabolic system further requires a functioning β-endorphin production system plays a further important role in the cellular clean-up system of the cells, thus being a further essential aspect for providing viable and vital cells and a functioning cellular defense system against harmful environmental influences.

Besides the aforementioned aspects of the metabolic system a further aspect relates to the melanogenesis of cells, i.e. the melanin production in the cells by melanocytes. Melanin is a pigment found in the skin, eyes, and hair, thus being responsible i.a. for the skin color. Melanocytes are located in the basal layer of epidermis. the. Melanocytes are dendritic cells connected to the keratinocytes in which they transmit specific organelles, melanosomes, containing melanin. Melanogenesis comprises two steps: melanin synthesis and then transfer to keratinocytes. Melanin is synthesized in the melanosomes of melanocytes, organelles that come from the Golgi apparatus and rough endoplasmic reticulum. The synthesis of melanin is made from the amino acid tyrosine, catalyzed by the tyrosinase enzyme. Tyrosine reacts to Dihydroxyphenylalanine (DOPA), which is then oxidized to Dopaquinon. After several further chemical reaction steps, finally melanin is formed. Within the melanocytes, the melanin pigment is trapped in "bags", the melanosomes that are then sent to the surface. The melanocytes have dendritic extensions allowing them to come into contact with several keratinocytes. The melanosomes are transported from the cell body where they are produced to the end of the dendrites where they accumulate and are transferred to adjacent keratinocytes where melanin is dispersed. Usually, melanogenesis leads to a long-lasting pigmentation, which is in contrast to the pigmentation that originates from oxidation of already-existing melanin. There are both basal and activated levels of melanogenesis. In general, lighter-skinned people have low basal levels of melanogenesis. Exposure to UV-B radiation causes an increased melanogenesis. The purpose of the melanogenesis is to protect the hypodermis, the layer under the skin, from the UV-B light that can damage it (DNA photodamage). The color of the melanin is dark, allowing it to absorb a majority of the UV-B light and block it from passing through this skin layer. Numerous stimuli are able to alter melanogenesis, or the production of melanin by cultured melanocytes, although the method by which it works is not fully understood. Also harmful environmental influences such as toxic pollutants and UV irradiation may trigger melanogenesis and, in turn, pigmentation. With regard to its protective function, a functioning melanogenesis desirably provides a constant and uniform pigmentation of the skin. Due to harmful environmental influences the melanogenesis of vital cells may be deteriorated and lead to the formation of undesired spot-like pigmentation and also to the formation of melanoma (also known as malignant melanoma), which is the most dangerous type of skin cancer and that develops from the pigment-containing melanocytes. The primary cause of melanoma is ultraviolet light (UV) exposure in those with low levels of skin pigment. Therefrom, it becomes apparent, that a functioning cellular metabolic system further requires a vital and functioning melanogenesis system, leading to a constant and uniform protective pigmentation of skin and avoiding undesired spot-like pigmentation and in particular the formation of melanoma. Thus, a functioning melanogenesis plays a further important role in the cellular clean-up system of the cells, thus being a further essential aspect for providing viable and vital cells and a functioning cellular defense system against harmful environmental influences.

From the aforesaid explanations it is in particular apparent that vital and functioning cells require a strong cellular clean-up system for a vital and functioning cellular defense system against harmful environmental influences, wherein such cellular clean-up system is in particular based on a vital and functioning cellular metabolic system with an optimal interaction of the key elements of functioning mitochondria, lysosomes, IL-8 production, β-endorphin production, melanogenesis and a strong and intact cellular transmembrane system, which according to the present invention together form the cellular clean-up system of the cells.

The inventors of the present invention have surprisingly found, that *Hedychium* or an extract thereof, as defined herein, is particularly suitable in the cosmetic use for the protection against harmful environmental influences and the protection against and/or treatment of environmental damages deriving therefrom by protecting or activating at least one of the cellular metabolic system and/or the intracellular barrier system (cellular transmembrane system).

In particular, the inventors of the present invention have surprisingly found, that *Hedychium* or an extract thereof, as defined herein, is particularly suitable in the cosmetic use for the protection against harmful environmental influences and the protection against and/or treatment of environmental damages deriving therefrom by protecting or activating at least one of the cellular clean-up system, as defined above, selected from

- mitochondrial protection and/or activation,
- lysosomal protection and/or activation,
- inhibition of cellular IL-8 release,
- cellular production of β-endorphin,
- inhibition of irregular melanin release, and

- protection and/or activation of the cellular transmembrane system by claudin restoration.

In a further aspect, the inventors of the present invention have surprisingly found, that *Hedychium* or an extract thereof, as defined herein, is particularly suitable in the cosmetic use for the protection against harmful environmental influences and the protection against and/or treatment of environmental damages deriving therefrom by protecting or activating at least one of the cellular clean-up system, as defined above, selected from

- mitochondrial protection and/or activation,
- lysosomal protection and/or activation, and
- protection and/or activation of the cellular transmembrane system by claudin restoration.

In a further aspect, the inventors of the present invention have surprisingly found, that *Hedychium* or an extract thereof, as defined herein, is particularly suitable in the cosmetic use for the protection against harmful environmental influences and the protection against and/or treatment of environmental damages deriving therefrom by protecting or activating at least one of the cellular metabolic system, as defined above, comprising in particular

- mitochondrial protection and/or activation,
- lysosomal protection and/or activation,
- inhibition of cellular IL-8 release,
- cellular production of β-endorphin and
- inhibition of irregular melanin release.

In a further aspect, the inventors of the present invention have surprisingly found, that *Hedychium* or an extract thereof, as defined herein, is particularly suitable in the cosmetic use for the protection against harmful environmental influences and the protection against and/or treatment of environmental damages deriving therefrom by protecting or activating at least one of the cellular metabolic system, as defined above, comprising in particular

- mitochondrial protection and/or activation, and
- lysosomal protection and/or activation.

In a further aspect, the inventors of the present invention have surprisingly found, that *Hedychium* or an extract thereof, as defined herein, is particularly suitable in the cosmetic use for the protection against harmful environmental influences and the protection against and/or treatment of environmental damages deriving therefrom by protecting or activating at least one of the cellular metabolic system, as defined above, comprising in particular

- inhibition of cellular IL-8 release,
- cellular production of β-endorphin and
- inhibition of irregular melanin release;

preferably inhibition of cellular IL-8 release and/or cellular production of β-endorphin.
In a further aspect, the inventors of the present invention have surprisingly found, that *Hedychium* or an extract thereof, as defined herein, is particularly suitable in the cosmetic use for the protection against harmful environmental influences and the protection against and/or treatment of environmental damages deriving therefrom by protecting or activating at least one of the intracellular barrier system / cellular transmembrane system, comprising in particular protection, increase (improvement) and/or regulation of tight junctions, disturbed calcium flux and calcium gradient, in particular by acting on the claudin restoration.
In a further aspect, the inventors of the present invention have surprisingly found, that *Hedychium* or an extract thereof, as defined herein, is particularly suitable in the cosmetic use for the protection against harmful environmental influences and the protection against and/or treatment of environmental damages deriving therefrom by protecting or activating at least one of

- the intracellular barrier system / cellular transmembrane system, such as by claudin restoration, and
- the cellular inflammation defense system, such as by inhibition of cellular IL-8 release and/or cellular production of β-endorphin.

In a further aspect, the inventors of the present invention have surprisingly found, that *Hedychium* or an extract thereof, as defined herein, such as in particular a *Hedychium coronarium* J. Koenig root extract, is particularly suitable in the cosmetic use for the protection against harmful environmental influences and the protection against and/or treatment of environmental damages deriving therefrom, which are selected from oxidative processes, comprising for example UV induced oxidative damages and oxidative stress, such as oxidative and/or nitrosative damages deriving from UV irradiation and environmental pollutants or toxins, by protecting and/or activating the cellular antioxidant and radical oxygen species (ROS) defense systems. The inventors have in particular found the suitability of *Hedychium* or an extract thereof, as defined herein, for the cosmetic use of protection against and/or treatment of oxidative stress (UV or pollutant induced) and/or oxidative damages deriving therefrom by such as in particular by reduction or inhibition of ROS liberation (ROS production).

Preferably, the present invention relates to the cosmetic use of *Hedychium* or an extract thereof, as defined herein, according to at least one of the mechanisms as described herein according to the methods and tests as described in the Examples below.

In the sense of the present invention cosmetic activity of *Hedychium* or an extract thereof in any of the cosmetic indications or underlying mechanisms described herein is meant to be present if an improvement compared to an untreated control can be observed, such as in particular if an improvement compared to an untreated control can be observed in any of the methods and tests as described in the Examples below.

Accordingly, a further aspect of the present invention relates to a cosmetic composition comprising *Hedychium* or an extract thereof, as defined herein, such as in particular a *Hedychium coronarium* J. Koenig root extract.

The cosmetic composition of the present invention may further comprise at least one cosmetically acceptable solvent and/or at least one auxiliary substance.

Cosmetically acceptable solvents comprise the solvents mentioned above with respect to the *Hedychium* extract. Particularly preferred are hydrophilic or water-miscible cosmetically acceptable solvents, such as in particular those mentioned above for the extract.

Cosmetically acceptable auxiliary substances include:

pH-adjusting agents such as buffer substances;
inorganic and organic acids or bases comprising all cosmetically acceptable inorganic and organic acids, which are well known to a person skilled in the art;
fat substances comprising

- mineral oils such as paraffin oils or vaseline oils, silicone oils, and
- plant-derived oils, such as coconut oil, sweet almond oil, apricot oil, maize oil, jojoba oil, olive oil, avocado oil, sesame oil, palm oil, eucalyptus oil, rosemary oil, lavender oil, pine oil, thyme oil, mint oil, cardamom oil, orange blossom oil, soya oil, bran oil, rice oil, rapeseed oil and castor oil, wheat germ oil and vitamin E isolated therefrom, evening primrose oil,
- plant lecithines (e.g. soya lecithin), sphingolipids/ceramides isolated from plants,
- animal oils or fats, such as tallow, lanolin, clarified butter, as well as
- neutral oil (Miglycol 812), squalane, fatty acid esters, esters of fatty alcohols, such as triglycerides, as well as
- the so-called basic cream ("Basiscreme") DAC which is an important basic composition according to the German Codex for medicaments (Deutscher Arzneimittel Codex) comprising glycerolmonostearat, cetylalkohol, medium-chained triglycerides, white vaseline, macrogol-1000-glycerolmonostearat, propylenglycol, and purified water, and
- waxes having a melting point corresponding to skin temperature, comprising animal waxes, e.g. beeswax, and plant-derived waxes, e.g. carnauba wax and candelilla wax, and mineral waxes, e.g. microcrystalline waxes, and synthetic waxes, e.g. polyethylene or silicone waxes, and in particular all oils and waxes suitable for cosmetic purposes (so-called cosmetic oils and waxes), such as mentioned, for example, in the CTFA publication Cosmetic Ingredient Handbook, 1st ed., 1988, The Cosmetic, Toiletry and Fragrance Association, Inc., Washington;

surface-active agents, such as dispersing agents, wetting agents, emulsifiers etc.;
fillers;
stabilizers;
cosolvents;
cosmetically common or other dyestuffs and pigments;
preservatives;
moisturizing agents;
antioxidants;
UV-protecting agents
softeners;
lubricants or slip agents;
skin-conditioning agents;

etc.

In principle any cosmetically acceptable auxiliary as described in particular cosmetic regulations and as approved for cosmetic applications as well as any suitable mixture be used. Cosmetically acceptable auxiliaries and mixtures thereof are well known to a person skilled in the art.

Preferred cosmetic auxiliaries are selected from the groups of antioxidants, moisturizing agents, softeners, skin-conditioning agents, fat substances, such as in particular cosmetic fats and oils, and UV-protecting agents.

Generally, the classification of the abovementioned substances into the category of auxiliary substances in the context of the present invention does not exclude the fact that these auxiliary substances may also exhibit certain cosmetic activity, which applies in particular to a certain degree to cosmetic oils and other auxiliaries with moisturizing and lipid replenishing effects.

The cosmetic composition of the present invention may further comprise at least one additional cosmetically active agent. In the context of the invention, cosmetic agents or agents prepared using cosmetic active composition are essentially agents in the sense of the German Food and Feed Code [German = LFGB], i.e. substances or formulations of substances which are intended for external use on humans for skin care agents, cleansing, care, or for influencing the appearance or the body odour, or for imparting odoriferous impressions, unless they are predominantly intended for alleviation or elimination of diseases, suffering, body damage or pathological symptoms.

Examples of cosmetically active agents generally include: antiacne agents, antimicrobial agents, antiperspirant agents, astringent agents, deodorizing agents, hair removal agents, conditioning agents for the skin, skin-smoothing agents, agents for increasing skin hydration, such as e.g. glycerol or urea, sunscreen agents, keratolytics, free radical scavengers for free radicals, antiseborrhoea agents, antidandruff agents, antiseptic active compounds, active compounds for treatment of signs of ageing of the skin and/or agents which modulate the differentiation and/or proliferation and/or pigmentation of the skin, vitamins, such as vitamin C (ascorbic acid) and its derivatives, such as, for example, glycosides, such as ascorbyl glucoside, or esters of ascorbic acid, such as sodium or magnesium ascorbyl phosphate or ascorbyl palmitate and stearate, L-ascorbic acid phosphate esters, alkali metal salts, such as sodium and potassium salts, of L-ascorbic acid phosphate esters; alkaline earth metal salts, such as magnesium and calcium salts, of L-ascorbic acid phosphate esters; trivalent metal salts, such as aluminium salts, of L-ascorbic acid phosphate esters; alkali metal salts of L-ascorbic acid sulfate esters, such as sodium and potassium salts of L-ascorbic acid sulfate esters; alkaline earth metal salts, such as magnesium and calcium salts, of L-ascorbic acid sulfate esters; trivalent metal salts, such as aluminium salts, of L-ascorbic acid sulfate esters; alkali metal salts, such as sodium and potassium salts, of L-ascorbic acid esters; alkaline earth metal salts, such as magnesium and calcium salts, of L-ascorbic acid esters; and trivalent metal salts, such as aluminium salts, of L-ascorbic acid esters, retinoids (retinol, retinal, retic acid), anthralins (dioxyanthranol), anthranoids, peroxides (in particular benzoyl peroxide), minoxidil, lithium salts, antimetabolites, vitamin D and its derivatives; catechols, flavonoids, ceramides, polyunsaturated fatty acids, essential fatty acids (e.g. gamma-linolenic acid), enzymes, coenzymes, enzyme inhibitors, hydrating agents, skin soothing agents, detergents or foam-forming agents, and inorganic or synthetic matting fillers, or decorative substances, such as pigments or dyestuffs and coloured particles for foundations, make-up formulations, and other agents for cosmetic adornment and coloured modelling of eyes, lips, face etc. and abrasive agents. Plant-derived active compound extracts or extracts or individual substances obtained therefrom (other than the *Hedychium* extract of the present invention) may furthermore be mentioned. Generally, the plant active compound extract may be selected from the group consisting of solid plant extracts, liquid plant extracts, hydrophilic plant extracts, lipophilic plant extracts, individual plant constituents; and mixtures thereof, such as flavonoids and their aglyca: rutin, quercetin, diosmin, hyperoside, (neo)hesperidin, hesperitin, Ginkgo biloba (e.g. ginkgoflavone glycosides), Crataegus extract (e.g. oligomeric procyanidins), buckwheat (e.g. rutin), Sophora japonica (e.g. rutin), birch leaves (e.g. quercetin glycosides, hyperoside and rutin), elder blossom (e.g. rutin), linden blossom (e.g. essential oil with quercetin and farnesol), St. John's wort oil (e.g. olive oil extract), Calendula, Arnica (e.g. oily extracts of the blossom with essential oil, polar extracts with flavonoids), Melissa (e.g. flavones, essential oil); immunostimulants: Echinacea purpurea (e.g. alcoholic extracts, fresh sap, pressed juice), Eleutherococcus senticosus; alkaloids: Rauwolfia (e.g. prajmalin), periwinkle (e.g. vincamin); further phytopharmaceuticals: Aloe, horse chestnut (e.g. aescin), garlic (e.g. garlic oil), pineapple (e.g. bromelains), ginseng (e.g. ginsenosides), Our Lady's thistle fruit (e.g. extract standardized with regard to silymarin), box holly root (e.g. ruscogenin), valerian (e.g. valepotriates, tct. Valerianae), kava kava (e.g. kava lactones), hop blossom (e.g. hop bitters), etr. Passiflorae, gentian (e.g. ethanol. extract), anthraquinone-containing drug extracts, e.g. aloin-containing Aloe vera juice, pollen extract, algae extracts, liquorice extracts, palm extract, Galphimia (e.g. original tincture) mistletoe (e.g. aqueous ethanol. extract), phytosterols (e.g. beta-sitosterol), verbascum (e.g. aqueous alcohol. extract), Drosera (e.g. vinum liquorosum extract), sea buckthorn fruit (e.g. juice obtained therefrom or sea buckthorn oil), marshmallow root, primula root extract, fresh plant extracts of mallow, comfrey, ivy, horsetail, yarrow, ribwort (e.g. pressed juice), stinging nettle, greater celandine, parsley; plant extracts from Norolaena lobata, Tagetes lucida, Teeoma siems, Momordica charantia, and Aloe vera extracts.

Preferred cosmetic active compounds are those being active in the use according to the present invention, such as in particular skin care agents, skin conditioning agents, skin-smoothing agents, agents for increasing skin hydration, such as e.g. glycerol or urea, sunscreen agents, keratolytics, free radical scavengers against free radicals, antiseborrhoea agents, active compounds for treatment of signs of ageing of the skin and/or agents which modulate the differentiation and/or proliferation and/or pigmentation of the skin.

The *Hedychium* extract or the cosmetic composition comprising the *Hedychium* extract according to the present invention is for topical application. Accordingly, the cosmetic composition according to the present invention is in the form of a topical composition. The topical compositions useful in the present invention involve formulations suitable for topical application to skin and may be made into a wide variety of product types that include, without being limited thereto, lotions, creams, gels, emulsions, sticks, sprays, ointments, cleansing liquid washes and solid bars, shampoos, pastes, mousses, wipes, patches, cosmetic dressings or masks and adhesive bandages, hydrogels, and films or al liposomal formulations.

The amount of the *Hedychium* extract present in the cosmetic composition will depend on the type of extract used and is generally selected be a safe and effective amount thereof. This means an amount *Hedychium* or the extract thereof as defined herein, which is sufficient to significantly induce a positive modification in the condition to be regulated or treated, such as in particular in the specific use as defined herein and very particularly according to the methods described in the Examples below, and which at the same time is low enough to avoid serious undesired side effects (at a reasonable benefit/risk ratio), within the scope of sound judgment of a skilled person. The safe and effective amount of *Hedychium* or the extract thereof may vary with the particular condition being treated, the age and physical condition of the user, the severity of the condition or impact being treated/prevented, the duration of the treatment, the nature of concurrent treatments or therapeutic therapies, the specific *Hedychium* species to be used, and may further depend in particular from the composition employed, i.e. the particular cosmetically-acceptable auxiliaries and/or additional cosmetically active agents utilized, and similar factors.

The *Hedychium* or the extract thereof typically will be present in the composition in an amount from about 0.001 % to about 20 % by weight, in particular in an amount from about 0.01 % to about 10 %, preferably in an amount from about 0.1 % to about 5.0 % by weight, more particularly in an amount from about 0.15 % to about 3.0 % by weight, even more preferably in an amount from about 0.2 % to about 2.0 % by weight, in each case based on the total weight of the cosmetic composition. Preferably in the cosmetic composition a liquid extract is used. Most preferred is a liquid extract of a concentration of 2 % (weight / volume).

Preferably, the cosmetic composition according to the present invention comprises an *Hedychium* extract as defined herein in an amount providing an equal concentration of the *Hedychium* extract based on the cosmetic composition in total as defined above for the *Hedychium* extract of the present invention. Then the respective concentrations will be determined in % (weight / volume) based on the total of a liquid cosmetic composition. In case of a non-liquid, i.e. semi-liquid, viscous, gel-like or creamy or even solid cosmetic composition equal concentrations will apply in % (weight / weight) based on the total of the non-liquid cosmetic composition.

In particular, the *Hedychium* extract or the cosmetic composition comprising the *Hedychium* extract according to the present invention is for any of the specific cosmetic uses as defined herein.

The present invention further relates to a process for preparing a *Hedychium* extract as defined herein, such as in particular a *Hedychium coronarium* J. Koenig root extract, the process comprising the steps of

(a) providing a certain amount of pieces of the *Hedychium* plant to be extracted,
(b) extracting the *Hedychium* plant pieces with a suitable solvent or solvent mixture,
(c) filtration of the extraction mixture of step (b),
(d) concentration of the filtrate of step (c) by settling of the solid residues and decantation of the supernatant,
(e) filtration of the supernatant of step (d) to obtain the *Hedychium* extract as a clear solution
(f) adjustment of the clear solution of step (e) to the desired concentration using a suitable solvent, such as preferably a mixture of water and glycerol, to obtain the final extract in the desired concentration.

In the process of the present invention in step (a) the pieces of the *Hedychium* plant are preferably selected from crushed, cut or milled roots and/or rhizomes of *Hedychium coronarium* J. Koenig.

In the process of the present invention in step (b) the solvent or solvent mixture is preferably a hydrophilic or water-miscible solvent, such as preferably selected from the solvents as described above. Preferably ethanol is used for extraction of the *Hedychium* pieces in step (b). Preferably one or more hydroalcoholic extractions (preferably using ethanol) are carried out. Preferably the extraction is carried out using plant pieces and hydroalcoholic solvent in a ratio 1 : 10 (e.g. 1 kg of plant pieces, such as in particular root/rhizome pieces extracted with 10 liters of 50% (w/w) ethanol.

In step (c) conventional filtration techniques are applied, which are well known to a person skilled in the art.

In step (d) the concentration by settling of the solid residues and decantation of the supernatant is carried out using conventional techniques, which are well known to a person skilled in the art.

In step (e) filtration of the supernatant is carried out for further clarification and purification of the supernatant to obtain the *Hedychium* extract in the form of a clear solution. Therein, filtration techniques are applied, which are well known to a person skilled in the art. Therein, the solvent is preferably as water glycerol mixture.

The adjustment of the clear solution to the desired concentration in step (f) is preferably carried out with a mixture of water and glycerol, to obtain the final extract in the desired concentration, in a solvent mixture having the ratio of water to glycerol of 30 : 70 (v/v).

The process may comprise further purification, concentration or refining steps, which lie within the common knowledge of a person skilled in the art.

The resulting *Hedychium* extract may be used for preparing a cosmetic composition as defined herein by applying conventional techniques for preparing the desired cosmetic application form.

The invention is further illustrated by the following examples, which relate to certain specific embodiments of the present invention. The examples were carried out using well known standard techniques within the routine to those of skill in the art, unless indicated otherwise. The following examples are for illustrative purposes only and do not purport to be wholly definitive as to conditions or scope of the invention. As such, they should not be construed in any way as limiting the scope of the present invention.

## DESCRIPTION OF THE FIGURES

Fig. 1 shows the results regarding mitochondrial protection of Example 2 with ATP dosage in fibroblasts treated for 48h with HCR extract (with irradiation or not).

Fig. 2 shows the results regarding mitochondrial protection of Example 2 with NAD/NADH dosage in fibroblasts treated for 48h with HCR extract (with irradiation or not).

Fig. 3 shows the results regarding lysosomal protection of Example 3 with quantification of LysoTracker fibroblasts staining after treatment for 48h with HCR extract (with irradiation or not).

Fig. 4 shows the results regarding IL-8 released by skin explants, pre-treated or not for 24h with HCR extract, polluted with BaP and re-treated for 24h of Example 4 .

Fig. 5 shows the results regarding dosage of $\beta$-endorphins released by keratinocytes after treatment for 40h with HCR extract of Example 5.

Fig. 6 shows the results regarding dosage of total proteins amount in keratinocytes after treatment for 40h with HCR extract of Example 5.

Fig. 7 shows the results regarding dosage of $\beta$-endorphins released by keratinocytes after treatment for 40h with HCR extract reported to total protein of Example 5.

Fig. 8 shows the results regarding dosage of released melanin by B16 melanocytes after treatment for 72h with HCR extract concentrations 0.001, 0.005 and 0.01 % of DE of Example 6.

Fig. 9 shows the results regarding dosage of released melanin by B16 melanocytes after treatment for 72h with HCR extract concentrations 0.01, 0.05 and 0.1 % of DE of Example 6.

Fig. 10 shows the results regarding dosage of total proteins amount in B16 melanocytes after treatment for 72h with HCR extract concentrations 0.001, 0.005 and 0.01 % of DE of Example 6.

Fig. 11 shows the results regarding dosage of total proteins amount in B16 melanocytes after treatment for 72h with HCR extract concentrations 0.01, 0.05 and 0.1 % of DE of Example 6.

Fig. 12 shows the results regarding dosage of released melanin by B16 melanocytes after treatment for 72h with HCR extract concentrations 0.001, 0.005 and 0.01 % of DE reported to total protein of Example 6.

Fig. 13 shows the results regarding dosage of released melanin by B16 melanocytes after treatment for 72h with HCR extract concentrations 0.01, 0.05 and 0.1 % of DE reported to total protein of Example 6.

Fig. 14 shows the results regarding the effects of HCR extract on ROS liberation in keratinocytes after 24h of treatment without ROS production stimulation of Example 7.

Fig. 15 shows the results regarding the effects of HCR extract on ROS liberation in keratinocytes after 24h of treatment with ROS production stimulation of Example 7.

## EXAMPLES

### Example 1 Preparation of *Hedychium coronarium* J. Koenig root extract

A *Hedychium coronarium* J. Koenig root extract is prepared according to the process of the present invention.

In step (a) crushed roots (rhizomes) of *Hedychium coronarium* J. Koenig, the root pieces having a size of up to 10 cm length, are provided in a suitable extraction vessel.

In step (b) 50 % (w/w) ethanol is added as the extraction solvent to the crushed roots and 2 extractions are carried out.

In step (c) the extraction mixture is filtered using conventional filtering techniques.

In step (d) the filtrate of step (c) is concentrated by settling of the solid residues, followed by decantation of the supernatant.

In step (e) the supernatant of step (d) is filtered using conventional filtering techniques. The *Hedychium coronarium* J. Koenig root extract is obtained as a clear solution.

In step (f) adjustment of the clear solution of step (e) is carried out to a solution of 20 g / liter in a mixture of water and glycerol having the ratio of 30 : 70 (v/v), followed by final filtration for further purification to obtain the final extract in the desired concentration of 2.0 % (w/v).

**Example 2 Evaluation of the effects of *Hedychium coronarium* J. Koenig root extract (HCR extracts) on mitochondrial network in fibroblasts irradiated with UVA (MitoTracker staining, ATP and NAD+/NADH dosages)**

1. Background Information

The aim of this study was to evaluate HCR extract effects on mitochondria distribution and cell energy metabolism of UVA-irradiated fibroblasts.

These cells (normal human fibroblasts, or NHF, from skin dermis) were cultured as primary monolayers; the test extract was applied during 24 hours, cells were irradiated (without treatment) and the test compound was re-applied during another 24 hours. Then mitochondria were stained, or cells were collected to perform ATP (adenosine triphosphate) and NAD+/NADH (nicotinamide adenine dinucleotide) dosages.

Biological Samples

Cells used in this study are primary cultures of normal human fibroblasts (NHF) extracted after plastic surgery from mammary skin dermis of a 41 year-old donor (Caucasian woman).

2. Dose Determination (Cytotoxicity/Viability Check by XTT Assay) and Evaluated Test Compound

After evaluation of the working doses by checking cell viability/toxicity with XTT assay a test concentration of 2 % (of dry extract / DE, corresponding to a dilution of 1:20 HCR in culture medium to finally obtain 0.1 % HCR) *Hedychium coronarium* J. Koenig root extract (HCR extract) has been selected.

Cell Seeding and Treatment

Cells were seeded into 96-wells microplates (at a cell density of 5,000 cells per well, i.e. 15,000 NHF/cm$^2$, the density used later for dosages), 24 hours before treatment (incubation at 37°C/5% CO 2).

Each concentration of the tested in sextuplicate. Incubation/treatment time is 48 hours (at 37°C/5% CO$_2$). Cells were seeded in complete medium: Dulbecco's modified Eagle medium or DMEM (Gibco 41966), with antibiotics (Gibco 15140) and 10% of foetal bovine serum or FBS (Gibco 10270). Treatments were done in medium without FBS.

Cytotoxicity Assessment (XTT)

After 48 hours of treatment, wells were emptied and gently rinsed with PBS (Phosphate Buffer Saline). A XTT solution (0.3 mg/mL) was then applied on cells and the plate was then incubated at 37°C/5% CO$_2$ in dark. After 3h of incubation, absorbance was measured at 450 nm with 650 nm reference. Blanks were also realized, with XTT solution in wells without cells (but with product or not), in order to verify absence of interaction between XTT molecule and the extract, that would be a bias in the results. The XTT test was performed using the kit "Cell Proliferation Kit II (XTT)" (Roche Diagnostics 11465015001).

The XTT system, a colorimetric method, is an assay to quantify mitochondrial activity and was used as a viability test. This test is based on the cleavage of yellow tetrazolium salt XTT to orange formazan, by the system "succinate-tetrazolium reductase" present in the mitochondrial respiratory chain of cells. Thus, this conversion occurs only in metabolically active cells, that means alive cells. The derivative formazan is measured spectrophotometrically at 450 nm with 650 nm reference.

Results Expression

For each condition, means of optic density data (OD, or absorbance) were calculated.

Viability of treated cells is expressed as percentage of untreated control

$$\% \ viability \ _{\text{"sample"}} = \frac{OD \ _{\text{"sample"}}}{OD \ Mean \ _{\text{"untreated control"}}} \times 100$$

A treatment that diminishes cells viability, below the 80% limit of mitochondrial activity compared to untreated control, was considered as cytotoxic for cells. On the contrary, an increase of data detects mitochondrial activity.

3 Evaluation of Mitochondrial Network Protection

Assay Principle

Fibroblasts were seeded in Petri dishes, afterwards pre-treated with 3 concentrations of the test compound during 24 hours, then UVA-irradiated and finally the test compound was applied again for another 24h. MitoTracker staining was then performed to observe mitochondria.
As a positive control of UV protection Vitamin C (500 $\mu$M) was used.

Cells Seeding and Treatments

Cells were seeded into Petri dishes (at a cell density of 150,000 cells per dish, with a diameter of 60mm, i.e. 7,700 NHF/cm$^2$), in the same medium as previously described (complete medium, i.e. with FBS), one day before treatment (incubation at 37°C/5% CO$_2$). Each test compound concentration/dose (in one dish each) was then applied for 24h (in medium without sera; incubation at 37°C/5% CO$_2$).
Then cells were UVA-irradited or not in a saline buffer (8 J/cm$^2$, in a Vilber Lourmat RMX 3W machine) and finally treated again with the test compound (in culture media) for another 24h.

Mitochondria Network Staining

To proceed to the mitochondria staining, cells were incubated with a fluorescent probe (Invitrogen MitoTracker ®). Cells were observed and photos were taken with x40 objective (Olympus CK40 microscope and Archimed Microvision software), with or without appropriate fluorescent filter. Then photos permited to visualize the mitochondria network or whole cells, respectively.

4. Evaluation Of Effect On Metabolic Activity (ATP & NAD+/NADH Dosages)

In order to verify effects of UV and tested product on cells metabolism, ATP and NAD+/NADH dosages were performed.

Assay Principle

The protocol was the same as for the previous assay, but with cells seeded in 96-wells plates: treatment for 24h, UVA-irradiation and finally application again for 24h. ATP and NAD+/NADH dosages were then performed (in cells lysates or permeabilized cells pellets).

Cells Seeding and Treatments

Cells were seeded into 96-wells plates (at a cell density of 5,000 cells per well, i.e. 15,000 NHF/cm$^2$), in the same medium as previously described, one day before treatment. Then the treatment and irradiation timing was the same: first application for 24h (in medium without sera), then UVA-irradiation or not (same dose) and finally a second treatment for another 24h. As previously, from beginning to end of the experiment, cells were incubated at 37°C/5% CO$_2$. Here each dose was tested in quintuplicate (for ATP) or in sextuplicate (for NAD+/NADH).

ATP and NAD+/NADH Dosages

In order to proceed to the dosages, cells lysates or pellets were collected at the end of treatment:

- For dosage of cellular ATP total level, detergent was added in wells containing cells and supernatants (media with product) to lyse cells and obtain cells lysates.

- For intracellular NAD+ and NADH dosage, supernatants were eliminated and cells were permeabilized to obtain permeabilized cells pellets.

Then dosages were performed with commercial kits:

luminescent ATP detection assay kit (Abcam ab113849) and NAD +/NADH cell-based assay kit (Cayman Chemical 600480).

The NAD assay kit doses the two existing forms (oxidized NAD+ and reduced NADH). Firstly, adding of ethanol and alcohol dehydrogenase permits to reduce NAD+ found in samples to NADH, concomitantly to oxidation of ethanol. Then total NADH is oxidized in the same time as the reduction of a tetrazolium salt substrate (WST-1) to a colored formazan. Measured quantity of this product (absorbance) is proportional to total NAD (NAD+ and NADH) in each sample.

The ATP assay kit is an ATP monitoring system based on firefly (Photinus pyralis) luciferase. Added luciferase and D-luciferin react with ATP in samples to produce light, which measure (luminescence) is proportional to the ATP concentration.

Results Expression

For both ATP and NAD+/NADH dosages, raw data, i.e. luminescence units (RLU) or OD measurements obtained for respective standards were plotted on graphics to determine standard curves. Then, the concentrations of ATP or NAD+/NADH measured in the samples were determined. Quantitative values of each condition were averaged. Data were graphically presented as concentrations (nM for ATP or NAD+/NADH).

Results obtained for each condition can also be expressed relatively to the untreated control, with or without irradiation (set to 100%), or to the respective same condition without irradiation (evaluation of UVA effect):

$$\% \text{ « sample »} = (\text{Mean RLU or OD } _{\text{« sample »}} / \text{Mean RLU or OD } _{\text{« control irradiated or not »}}) \times 100$$

The statistically significant effects of the results were determined by the Student's t-test using the same criteria as described for the XTT assay (comparison to non- irradiated or irradiated control, or to the respective same condition without irradiation).

5. Results

Dose Determination (Cytotoxicity/Viability Check by XTT Assay)

The cytotoxicity dose determination assessment no cytotoxicity was observed for the tested concentrations between 0.0005% and 0.01% HCR (dry extract / DE) on fibroblasts:

| | *Concentrations to test (MitoTracker assay and metabolism dosages)* |
|---|---|
| **HCR** | 0.01 - 0.005 - 0.001 % (of DE) |

Evaluation of Mitochondrial Network Protection

Observation of images permitted to see effect of UV irradiation on cells shape and mitochondria distribution in cells. Indeed, some UV-irradiated fibroblasts were no more spindle-shaped and mitochondria network seemed less organized than without UV irradiation.

With application of the positive control (vitamin C, 500 μM), effects of UVA were less distinct. Treatment of fibroblasts with HCR at 0.01% (of DE) protected mitochondria network impaired by UV irradiation. This effect against UV has not been observed with the lower doses of 0.005 and 0.001%.

Evaluation of Effect on Metabolic Activity (ATP & NAD+/NADH Dosages)

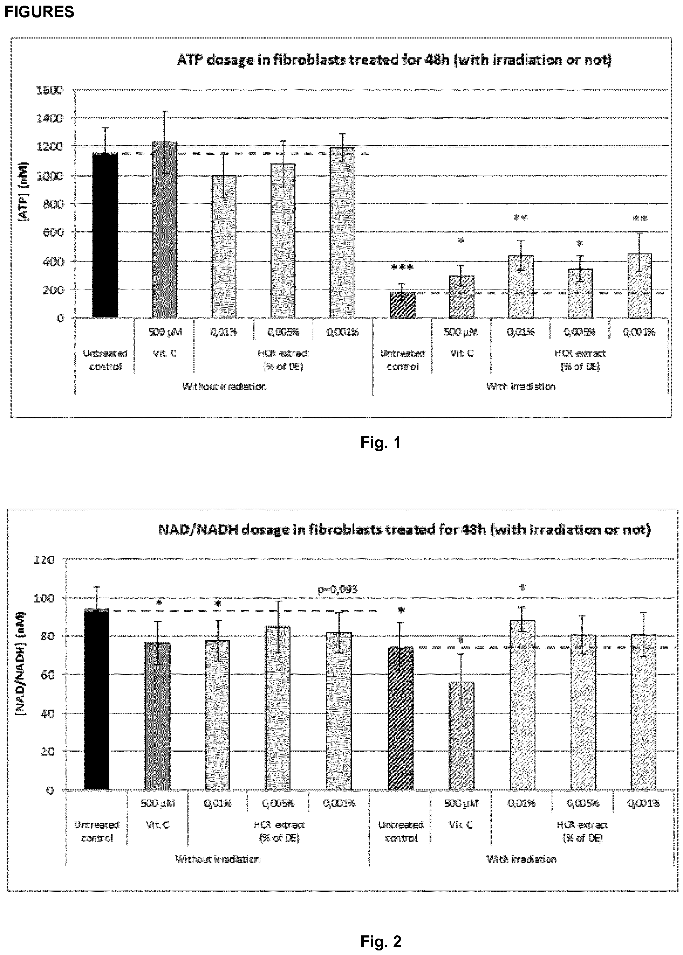

Results of dosages are presented in Figure 1. Data were expressed as concentrations (nM) reported to non-irradiated or irradiated control, or non-irradiated respective condition.

Statistics p-values are calculated in comparison to: non-irradiated control (black stars in Fig. 1), or irradiated control (red stars in Fig. 1).

Without irradiation, none of the treatments induced significant inhibition or stimulation of ATP production. UV irradiation induced a strong and very highly significant inhibition of ATP production by fibroblasts (-84 %***, irradiated control compared to basis level, i.e. without irradiation). The positive reference (vitamin C, 500 $\mu$M) permitted to counteract significantly UVA effect on this energy synthesis (+61%* compared to irradiated control). With HCR treatment, a significant or highly significant increase of ATP synthesis was observed: +136%**, +86%* and +146%** with 0.01, 0.005% and 0.001%, respectively.

NAD / NADH dosages

In the absence of irradiation, all treatments tended toward inhibition of basis NAD+/NADH fibroblastic production. The results are shown in Figure 2.

With UVA irradiation, this coenzyme synthesis by NHF was significantly inhibited (-21 %*, compared to basis level). Here vitamin C did not show expected positive effect on NAD+/NADH release by UV-irradiated cells. HCR application at the highest tested dose (0.01%) permitted to restore NAD+/NADH fibroblastic production decreased by UVA (+19%*, compared to irradiated control). At the two other tested doses, the effect was not significant (+9%).

In summary, the present evaluation shows that UVA induces partial deconstruction/disintegration of mitochondria network and inhibition of ATP and NAD+/NADH productions.

HCR extract shows effects on cultured and UV-irradiated fibroblasts:

- HCR increases significantly ATP production.
- HCR stimulates NAD+/NADH synthesis.
- the tested extract permits a protection of mitochondria network against UV irradiation.

Accordingly, this evaluation permits to conclude that HCR extract is efficient to protect skin against UV damage on mitochondria and energy metabolism.

**Example 3 Evaluation of the effects of *Hedychium coronarium* J. Koenig root extract (HCR extracts) on lysosomal network in fibroblasts irradiated with UVA (LysoTracker staining)**

1. Background Information

The aim of this study was to evaluate HCR extract effects on lysosomes distribution in UVA-irradiated fibroblasts.

These cells (normal human fibroblasts, or NHF, from skin dermis) were cultured as primary monolayers; the test extract was applied during 24 hours, cells were irradiated (without treatment) and the test compound was re-applied during another 24 hours. Then lysosomes were stained with a specific test compound.

Biological Samples

Cells used in this study are primary cultures of normal human fibroblasts (NHF) extracted after plastic surgery from mammary skin dermis of a 41 year-old donor (Caucasian woman).

2. Dose Determination (Cytotoxicity/Viability Check by XTT Assay) and Evaluated Test Compound

The dose determination with XTT assay has been carried out as described in Example 2.

3. Evaluation of Lysosomes Network Protection

Assay Principle

Fibroblasts were seeded in Petri dishes, afterwards pre-treated with 3 concentrations of the test compound during 24 hours, then UVA-irradiated and finally the test compound was applied again for another 24h. LysoTracker staining was then performed to observe lysosomes.

As a positive control of UV protection Vitamin C (500 $\mu$M) was used.

Cells Seeding and Treatments

Cell seeding was carried out as described in Example 2.

Lysosomes Network Staining and Quantification

To proceed to the lysosomes staining, cells were incubated with a fluorescent probe (Invitrogen LysoTracker®). Cells were observed and photos were taken with x40 objective (Olympus CK40 microscope and Archimed Microvision software), with or without appropriate fluorescent filter. Then photos permited to visualize the lysosomes network or whole cells, respectively.
Fluorescence was quantified with Colombus software, by HCS Pharma (Rennes, France), as mean intensity (3 images for each condition). It is the mean value of intensity for each pixel of staining, contained in the cell area stained with LysoTracker (number of cells of each image is taken into account, as the final value is the mean of all cells values on the image).

4. Results

Dose Determination (Cytotoxicity/Viability Check by XTT Assay)

The cytotoxicity dose determination assessment no cytotoxicity was observed for the tested concentrations between 0.0005% and 0.01% HCR (dry extract / DE) on fibroblasts:

|  | *Concentrations to test (LysoTracker assay)* |
|---|---|
| **HCR** | 0.01 - 0.005 - 0.001 % (of DE) |

Evaluation of Lysosomal Network Protection

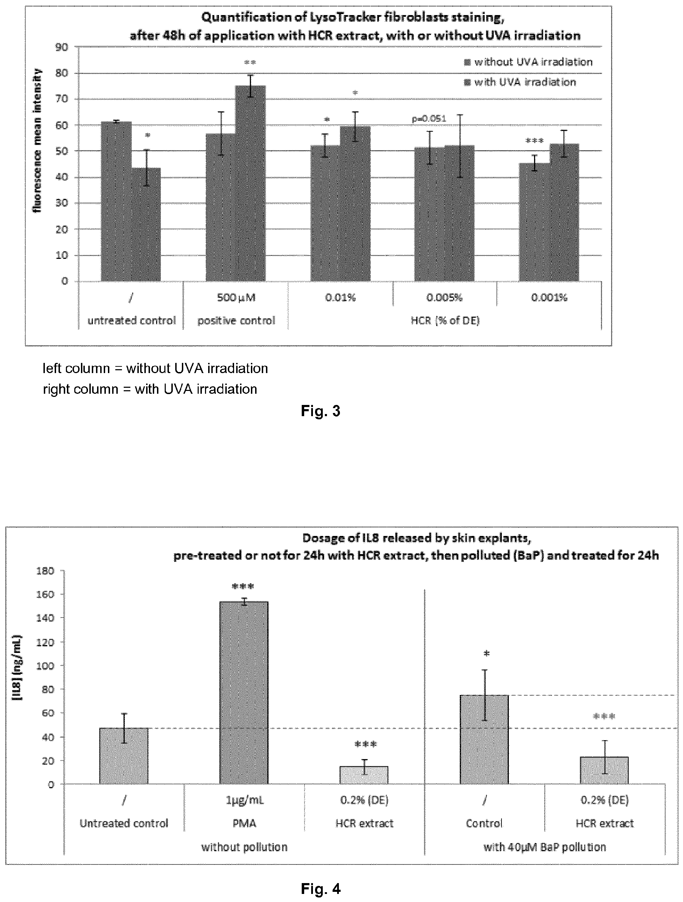

Observation of UV-irradiated control images permitted to see effect of irradiation on cells shape and lysosomes organization in cells. Some UV-irradiated fibroblasts were indeed no more spindle-shaped and lysosomes network seemed less organized, compared to untreated and non-irradiated control.
Quantification corroborated this effect, as fluorescence intensity decreased: -29% (significant *). Application of positive control (vitamin C, 500 $\mu$M) diminished effects of UVA. The results are shown in Figure 3.
Quantification data supported this observation, as fluorescence mean intensity was higher (highly significant effect **).
Treatment of fibroblasts with HCR at 0.01% protected lysosomes network damaged by UV irradiation. This effect against UV was less distinct with 0.005 and 0.001% of extract.
This is confirmed by results of fluorescence quantification, as there was an increase of +36% with 0.01% HCR (significant *, compared to irradiated control). With lower tested doses (0.005% and 0.001%), effects were less distinct and not significant (+19% and +21 % respectively).
Nevertheless, it has to be noted that observation is an important criteria here, indeed quantification is not the only endpoint that has to be considered, because some effects are seen by observation but are not highlighted with quantification: qualitative criteria, as network organization and cell shape.
In summary, the present evaluation shows, that UVA irradiation causes partial damage of cell lysosomal network.
In conclusion, tested HCR extract show effects on UV-irradiated fibroblasts and permits a protection of network against UV irradiation, particularly at highest tested dose (0.01%).
Accordingly, this evaluation permits to conclude that HCR extract is efficient to protect of dermis cells against UV damage.

**Example 4 Evaluation of the effects of *Hedychium coronarium* J. Koenig root extract (HCR extracts) in a human skin explants pollution model**

1. Background Information

The aim of this study was to assess the potential protective effect of HCR extract, in a pollution model of human skin explants.
The product was systemically applied for 24 hours, and then applied again during pollution, for another 24 hours. Release of the inflammatory cytokine IL-8 (interleukin 8) by the explants was analyzed by dosage in the supernatants (culture media).

In addition, skin explants were preserved for analysis by immunostainings of claudins 1 & 4 expressions (CLDN1 et 4).

Pollution was induced in this model by application of an environmental pollutant, benzo(a)pyrene (3,4-benzopyrene/BaP) as systemic treatment, i.e. in the culture medium. This pollutant induces IL-8 release (SEPhRA results).

BaP is produced by incomplete combustion of animal fat in contact with charcoal flames, in fumes of Diesel motors in case of insufficient combustion, in tar vapors. A tobacco cigarette produces 18 to 50 ng of BaP.

Biological Samples

The human skin explants used in this study (diameter of 8 mm), were provided by the company Biopredic International (Rennes) and were derived from mammary plastic surgery (Caucasian origin 44 year-old woman)

2. Human Skin Explants Model of Pollution

Test Summary

A *Hedychium coronarium* J. Koenig root extract (HCR extract) in a concentration of 2 % (of dry extract / DE, corresponding to a dilution of 1:20 HCR in culture medium to finally obtain 0.1 % HCR) has been used as test compound.

Extract to be tested was systemically applied as a pre-treatment during 24h. Then, the skin explants were polluted or not with BaP (Sigma CRM40071, 40µM systemic) for another 24h, while extract was applied again (systemically for both).

Each non-polluted condition was tested in triplicates (3 explants: 2 for immunostainings and 1 for histology), each polluted condition was tested in quadruplicates (4 explants: 3 for immunostainings and 1 for histology).

Dosages in supernatants (removed for each condition, for the 3 non-polluted explants and the 4 polluted explants) were done in duplicates.

In order to validate the IL-8 dosage, an additional condition was evaluated, i.e. stimulation with a reference pro-inflammatory molecule PMA (Phorbol 12-myristate 13-acetate, Sigma P8139, 1 µg/mL systemic - 1 explant, not treated with the extract nor polluted). This product was applied during the last 24h (instead of the pollutant).

3. Treatments of the Skin Explants

After delivery, skin explants were placed in 24-wells plates filled with DMEM medium (Invitrogen 31053) added with 5% foetal calf/bovine serum (FBS, Invitrogen 10270) and antibiotics (penicillin 100 U/mL and streptomycin 100 µg/mL, Invitrogen 15140) (300 µL/well). Explants were stabilized during 4 hours at 37°C/5 % $CO_2$.

After stabilization, extract was systemically applied or not in the medium of the explants, that were incubated at 37°C/5 % $CO_2$ for 24 hours. The medium used was then DMEM not added with FBS. After these 24 hours, explants were polluted or not with systemic application of BaP (40µM) for 24h. During this time, explants were treated again (or not) with the extract (37°C/5 % de $CO_2$ incubation).

At the end of the 24h+24h time, supernatants (treatment media) were stored in order to dose IL-8.

One explant of each condition (polluted or not) was preserved in 4% formaldehyde to perform the histological analysis (H/E/S staining). Other explants were cryopreserved in liquid azote, for later immunostaining experiments.

I L-8 Dosage

The inflammatory cytokine IL-8 was dosed with a commercial kit from Bio-Techne/R&Dsystems (D8000C), which is an ELISA method. For each condition, each triplicate/quadruplicate was dosed twice (duplicate). Dosage raw data were obtained by measuring absorbance or OD (optic densitiy) with 450 nm wavelength, with 570 nm as a reference.

Date Interpretation

The concentrations of IL-8 measured in the samples were determined by using a concentration range of an IL-8 standard. OD data obtained for IL-8 standards (pg/mL) were plotted in a standard curve with the concentrations on the abscissa axis and OD on the ordinate axis and using a calibration straight line IL-8 quantity (pg/mL or ng/mL) was determined from OD data by subtracting for each data the blank mean.

$$OD_{\text{« sample »}} = OD_{\text{raw data}} - OD\ \text{mean}_{\text{« blank »}}$$

Quantitative values of each condition were averaged and the IL-8 concentrations (ng/mL) are shown in Figure 4.

### 4. CLDN 1 and 4 Immunostainings

#### Test Principle and Immunostaining Protocol

In this study, expression of these proteins was observed by immunostaining. Fluorescent immunostaining aim is the detection of target protein with two antibodies: the first one, the primary antibody, is specific of the protein, so detects the protein. Then, a secondary antibody, specific of the primary antibody, permits the detection of the protein-primary antibody complex and emits fluorescence. This technique permits to observe the expression profile of the target protein in the target tissue, here epidermis skin.

Before immunostaining, the cryopreserved explants were cut with a cryostat. Frozen cross-sections were mounted on slides and cryopreserved. To start immunostaining, slides were defrosted and fixed with acetone. Primary antibodies non-specific fixation sites were then blocked ("blocking") with serum from the same species as the secondary antibody (Santa Cruz). After incubation at room temperature with the primary antibody (that target claudin 1 or 4, Santa Cruz sc-81796 or sc-17664 respectively), cross-sections were incubated with secondary antibody coupled to a fluorochrome (Alexa type, Invitrogen, 488 or 546 nm, green or red) and DAPI (Roche), a blue fluorescent marker specific to DNA, to color the nucleus.

Finally, slides were "mounted", with a mounting medium (Dako) that provides fluorescence protection and coverslips adhesion onto the slide. Slides were observed with x40 objective under a fluorescence optic microscope (Olympus CK40) with appropriate filters to the fluorochromes (FITC green, or TRITC red) or to DAPI (blue). Images acquisition was done with Archimed logiciel (Microvision).

For each of these two stainings, a specificity control of the primary antibody was done, isotypic control. On one supplementary cross-section slide, instead of the primary antibody, a serum of non-specific immunoglobulins was used, from the same species of the primary antibody (IgG, Santa Cruz).

#### Histology

A histology control was made to verify tissue integrity of skin explants, polluted or not, treated or not (check of the received tissues quality and absence of tissue structure deterioration by treatments). Explants were fixed and preserved in 4% formaldehyde at the end of treatment (one for each condition). Explants ere then included in paraffin, cross-sections were mounted on slides, and colored with hematoxyline/eosine/safran (or HES).

### 5. Results

#### Histological Analysis

This analysis permitted to check that after incubation time, explants of untreated condition presented a normal tissue structure (quality of this donor explants, during the experiment time and in the survival conditions). Furthermore, after treatment time, the test compound application did not damage integrity of tissue structure.

For claudin 1, its degradation (inhibition of expression and meshing failure) was observed in case of systemic application of the BaP pollutant.

HCR extract (0.2%, systemically applied) restored deteriorations caused by the pollutant.

For claudin 4, BaP pollutant degraded also expression of this claudin (architecture damage, decrease of fluorescence intensity: thinner and less thick areas), so modulated integrity of this cell tight junction.

Systemic application of HCR extract restored the meshing failure caused by the pollutant.

#### Dosage of IL-8 Released from Skin Explants

IL-8 dosage results after pollution or not, are shown in Figure 4. Therein, data are expressed as IL-8 concentrations. Significance is indicated, compared to the unpolluted control (black stars), or compared to the polluted control (red stars).

After pollution, the cytokine release was stimulated: +59% significant (*) (polluted control compared to the untreated/unpolluted control). With PMA stimulation (pro-inflammatory reference molecule), without pollution, the cytokine release was stimulated: +224% very highly significant (***) (compared to the untreated/unpolluted control).

With systemic application of HCR extract (0.2%), IL-8 release, major pro-inflammatory cytokine, was inhibited in both conditions: polluted or not. Without pollution, this effect was - 69% and very highly significant (***, compared to the untreated/unpolluted control). With pollution, IL-8 production, which was inducted by the pollutant, was inhibited by application of the HCR extract: -69%, very highly significantly (***, compared to the polluted control).

The results demonstrate the efficacy of the testedHCR extract to fight against an inflammatory stress mediator that is inducted by pollution.

In summary, the examined effects of the test compound were observed on the two endpoints evaluated:

- Inhibition of IL-8 release, induced or not by the pollutant, with the test compound being applied systemically.
- Restoration of claudins 1 and 4 expressions, damaged by the pollutant, by application of the test compound (systemic treatment).

Accordingly, this evaluation permits to conclude that HCR extract is efficient to protect skin of an inflammatory stress inducted by a pollutant, and of degradations undergone by proteins that participate to maintain tissue integrity, homeostasis and epidermis barrier function.

**Example 5 Evaluation of the effects of *Hedychium coronarium* J. Koenig root extract (HCR extracts) on $\beta$-endorphins production by normal human keratinocytes**

1. Background Information

The aim of this study was to evaluate in vitro effects of four HCR extracts on $\beta$-endorphins (or $\beta$-EP) by normal human keratinocytes (NHK), cultured as monolayer.

The $\beta$-endorphins are neuropeptides that were dosed in the culture supernatants by ELISA technique, after 40 hours of treatment. Previously, a XTT assay was performed, to check the cells viability, in order to determine the concentrations to be tested, as described in Example 2.

Biological Samples

Cells used in this study were normal human keratinocytes (NHKs), extracted from abdominal skin epidermis of a 30 year-old donor (Caucasian woman, number 5004).

2. Dose Determination (Cytotoxicity/Viability Check by XTT Assay) and Evaluated Test Compound

The dose determination with XTT assay has been carried out as described in Example 2, with the difference of 24 hours of treatment (instead of 48 hours).

Cells Seeding and Treatments

Cells were seeded at 12 500 keratinocytes/well (equivalent to 37 500 cells/cm$^2$, the cell density used later in the study), in 96-well microplates, in complete KSFM (Keratinocytes Serum Free Medium, with antibiotics and growth supplements), 24 hours before treatment (incubation at 37°C/5% $CO_2$). Each concentration of the test compound was tested in triplicate, in basal, i.e. non-supplemented medium (KSFM without growth supplements). Incubation time was 24 hours (at 37°C/5% $CO_2$).

3. $\beta$-Endorphins Production by Keratinocytes

Assay Principle

NHKs were seeded in 24 wells plates and treated with different concentrations of the test compound, during 40 hours. The $\beta$-EP were dosed in culture supernatants, by ELISA assay from USCN Life Science (CEA806Hu). Total proteins were also dosed, in cells pellets, in order to report $\beta$-EP dosage to total proteins amount.

dbAMPc (N6, 2'-O-Dibutyryladenosine 3',3'-cyclicmonophosphate, Sigma D0627) at 2mM was used as a positive control.

Cell Treatment and $\beta$-EP Assay

Cells were seeded at 75 000 cells/well (37 500 cells/cm$^2$), in 24 wells plates, in complete KSFM, 48 hours before treatment (incubation at 37 °C/5% $CO_2$). Each concentration of the test compound was tested in triplicate, in basal KSFM, added with an anti-proteases cocktail of molecules (Leupeptine, Aprotinin and Phenylmethanesulfonyl fluoride or PMSF, Sigma L8511, A1153 and P7626 respectively). This cocktail permitted to protect the β-EP proteins until the dosage. Incubation time was 40 hours (at 37 °C/5% $CO_2$).

Culture supernatants and cells pellets were collected and stored at -20°C before testing.

Absorbance data (or optical density, OD) were measured at 450 nm. Each sample was assayed in duplicate, so there were six values for each sample (except for untreated control and reference molecule, for which there were 8 and 2 values respectively).

Proteins Dosage (Bca Assay)

Total proteins dosage in cell pellets was performed in parallel by colorimetric method based on bicinchoninic acid. The principle of the bicinchoninic acid (BCA) assay is similar to the Lowry procedure, in that both rely on the formation of a protein-$Cu^{2+}$ complex under alkaline conditions, followed by reduction of the $Cu^{2+}$ to $Cu^+$. BCA is highly specific for $Cu^+$ ion and forms a purple-blue complex with $Cu^+$ in alkaline environments, thus providing a basis to monitor the reduction of alkaline $Cu^{2+}$ by proteins. The amount of reduction and of BCA-$Cu^+$ complex is proportional to the protein present and is quantified by spectrophotometric lecture (570nm longwave).

The proteins dosage kit used (Sigma BCA1) is composed of a bicinchoninic acid solution (Sigma B9643) and a copper sulfate pentahydrate solution ($CuSO_4$ - Sigma C2284). Standard range is prepared with BSA (Bovine Serum Albumin - Sigma A9418).

Cells pellets were dry preserved at -20°C awaiting cells lysis and dosage. To lyse cells and alkalinize the reactional environment, cells pellets were equilibrated at room temperature and then lysed in alkaline solution during 30 minutes. Dosage was realized adding a mix of the reagents (bicinchoninic acid + $CuSO_4$) to aliquots of lysates (cells pellets lysed). The plates were incubated at 37°C and then lecture was performed at 570 nm longwave, after having stopped the reaction placing the plates at 4°C for a few minutes.

Expression of Results

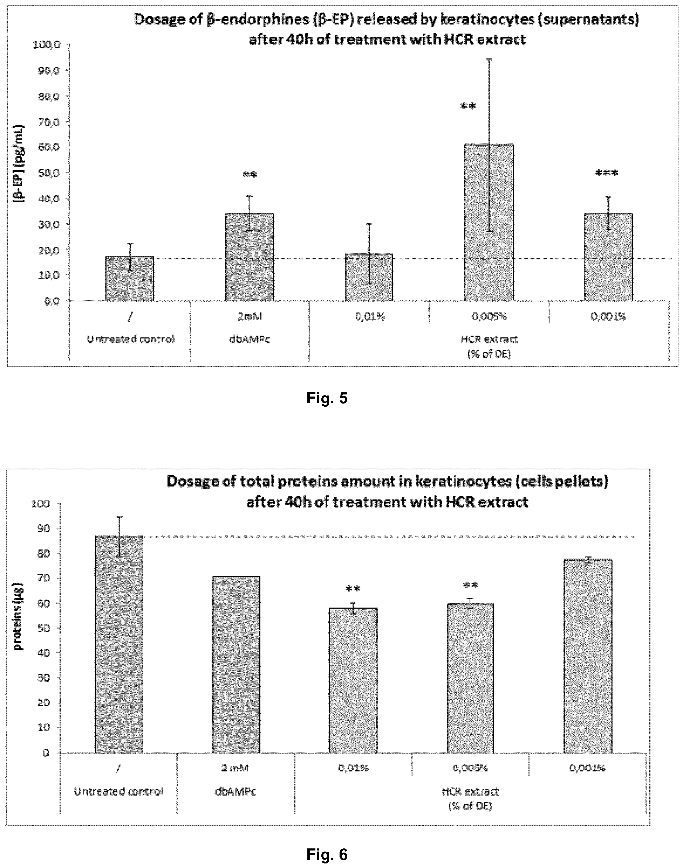

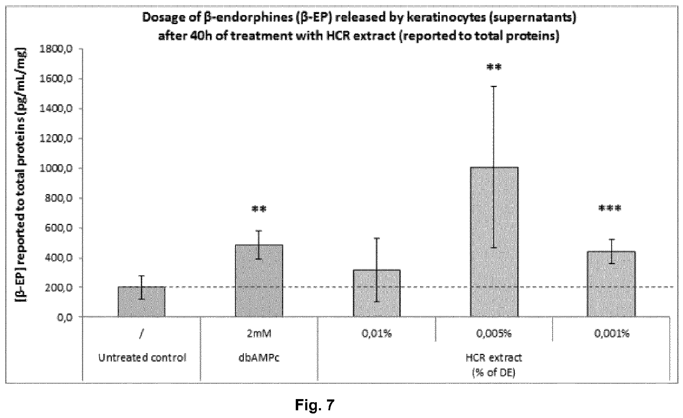

For both β-EP and proteins dosages, OD data obtained for respective standards ere plotted on graphics to determine standard curves. Then, the amount/concentrations of proteins or β-EP measured in the samples can be determined. Quantitative values of each condition were averaged. The results are shown in Figure 5, 6 and 7 with amount/concentrations (pg/mL for β-EP and μg/well for total proteins).

4. Results

Dose Determination (Cytotoxicity/Viability Check by XTT Assay)

The cytotoxicity dose determination assessment no cytotoxicity was observed for the tested concentrations between 0.0005% and 0.01% HCR (dry extract / DE) on fibroblasts:

| | *Concentrations to test (ß-EP) (chosen according to XTT assay)* |
|---|---|
| **HCR extract** | 0.01 - 0.005 - 0.001 % |

β-Endorphins Dosage

Treatment with HCR extract permits a beneficial effect on β-EP release by keratinocytes, as an induction appears with two tested concentrations: +102%*** at 0.001% and +259% ** at 0.005%. The results are shown in Figure 5.

Total Protein Dosage

Reported to total proteins amount, the beneficial effect on β-EP release by keratinocytes observed after treatment with HCR extract is corroborated, since a stimulation is highlighted with two tested concentrations: +120% *** at 0.001% and +402% ** at 0.005%.

In summary, in the present model of normal human keratinocytes, β-endorphin (β-rep) are produced and released in the culture supernatants in which the dosage is done. Treatment of normal human keratinocytes with HCR extract (0.001 and 0.005%) induced stimulation of β-EP release.

**Example 6 Evaluation of the effects of *Hedychium coronarium* J. Koenig root extract (HCR extracts) on melanogenesis in B16 cells (melanin dosage)**

1. Background Information

The aim of this study is to evaluate the effects of HCR extract on melanogenesis in an in vitro model of murine B16 melanocytes. These melanocytes were cultured as monolayers; the test compounds were applied during 72 hours and then melanin synthesized and released was dosed (in supernatants).

Previously, a XTT assay was performed, to check the cells viability, in order to determine the concentrations to be tested, as described in Example 2.

Biological Samples

Cells used in this study were murine melanocytes (B16-F0 line, LGC standards, ATCC-CRL-6322) extracted from murine skin. This cell line synthesizes a great quantity of melanin which is released in the culture media (so called supernatants), so the spectrophotometric dosage is easy.

2. Dose Determination (Cytotoxicity/Viability Check by XTT Assay) and Evaluated Test Compound

The dose determination with XTT assay has been carried out as described in Example 2, with the difference of 72 hours of treatment (instead of 48 hours).

Cells Seeding and Treatments

Cells were seeded into 96-wells microplates (at a cell density of 10,500 cells per well, i.e. 31,250 B16/cm$^2$, the density used later in the study), 24 hours before treatment (incubation at 37°C/5% $CO_2$). Each concentration of the test compound was tested in sextuplicate. Incubation/treatment time was 72 hours (at 37°C/5% $CO_2$). Cells ere seeded and treated in complete medium: Dulbecco's modified Eagle medium or DMEM, without phenol red (Gibco 11880), with antibiotics (Gibco 15140), L-glutamine (Gibco 25030) and supplemented with 10% of foetal bovine serum or FBS (Gibco 10270).

3. Evaluation of Effect on Melanogenesis

Assay Principle

B16 melanocytes were seeded in 24-wells plates. Then the cells were treated with the test compound (three concentrations), for 72 hours. Finally, melanin and total proteins dosages were performed.

Photos of cells culture every 24 hours permitted also to observe melanin release all along the extract application (for 72h).

Positive controls were used in this study:

a reference molecule inhibiting melanogenesis (tyrosinase inhibitor), kojic acid (Sigma K3125) at 1 mM, and at the contrary, a reference molecule that stimulates pigmentation (cAMP stimulator), an αMSH analog so called melanotan (Sigma M8764, [Nle4, D-Phe7]-α-Melanocyte Stimulating Hormone trifluoroacetate salt) at 100 nM.

Effects of these references were visible as soon as 48h of treatment for both and also at 72h for kojic acid. Three concentrations of the extract were tested (weight/volume % of dry extract or DE). These non-cytotoxic doses were tested according to the results obtained from the XTT assay.

Cell Treatment and Melanin Dosage

Cells were seeded into 24-wells plates (at a cell density of 60,000 cells per well, i.e. 31,250 B16/cm$^2$), in the same medium as previously described, one day before treatment (incubation at 37°C/5% $CO_2$). Each concentration/dose of the test compound was then tested in triplicate for 72h (incubation at 37°C/5% $CO_2$).

Coloration of culture media was observed every 24 hours and compared to blanks (wells with treatment media but without cells). After 48 or 72 hours of treatment, the supernatants were homogenized and aliquots were transferred to 96-well plates for spectrophotometry reading at 492 nm wavelength (reading in duplicate).

This dosage permitted to quantify melanin liberated by the B16 cells, relatively to a standard range of synthetic melanin (M0418).

Total Protein Dosage (Bca Assay)

Total proteins dosage in cell pellets was performed in parallel by colorimetric method based on bicinchoninic acid as described in Example 5.

Expression of Results

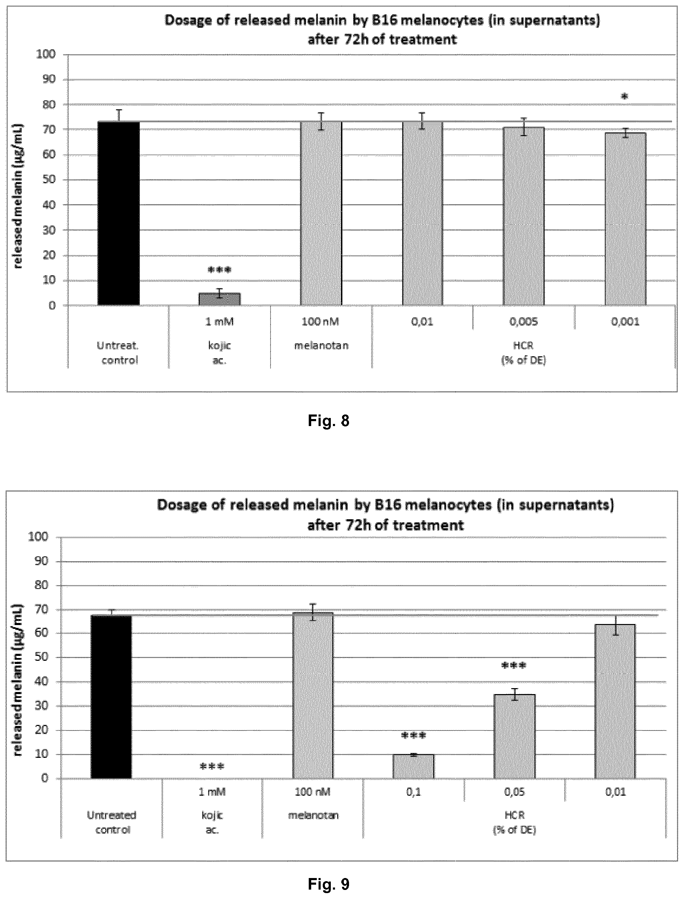

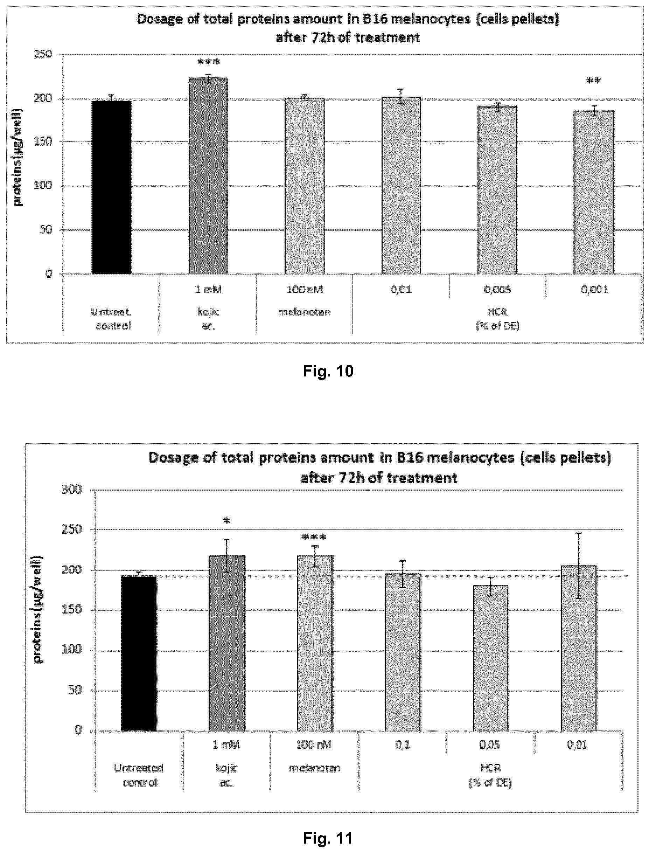

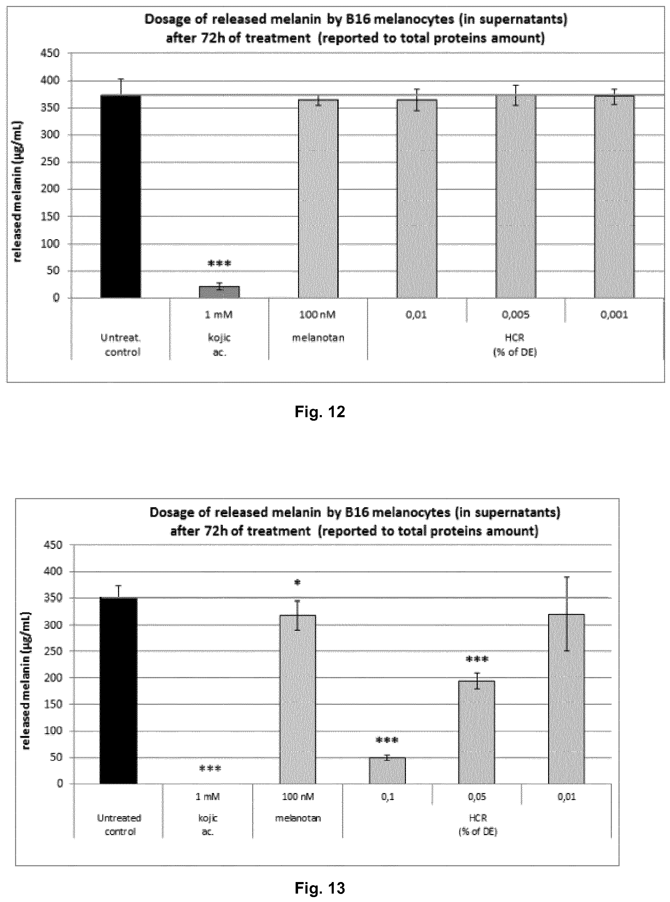

For both melanin and proteins dosages, OD measurements obtained for respective standards were plotted on graphics to determine standard curves. Then, the amount/concentrations of proteins or melanin measured in the samples can be determined. Quantitative values of each condition were averaged. The results with different concentrations of HCR extract are presented in Figures 8 to 13

4. Results

Dose Determination (Cytotoxicity/Viability Check by XTT Assay)

The cytotoxicity dose determination assessment no cytotoxicity was observed for the tested concentrations between 0.0005% and 0.01% HCR (dry extract / DE) on fibroblasts:

| | *Concentrations to test (melanogenesis)* | |
|---|---|---|
| **HCR (% of DE)** | 1st experiment | 0.01 - 0.005 - 0.001 % |
| | 2nd experiment | 0.1 - 0,05 - 0.01 % |

Melanin Dosage

In these experiments, the positive reference of depigmentation kojic acid (1 mM) induces a very strong and very highly significant (***) inhibition of the melanin release in the culture medium (more than 90%). After 48h of treatment, the positive reference of pro-pigmentation melanotan (100 nM) induces a strong and highly significant (**) stimulation of the melanin release in the culture medium (more than two fold) (Results not shown in detail).

At 72h the melanine release is more distinct than at 48h (OD more than 20-fold higher), which is normal and permits to observe potential pro-pigmentation effects at 48h.

The tested HCR extracts showed distinct depigmentation effects with the two highest tested concentrations after 72h of treatment in this model. After 72h of application in this model, HCR extract inhibited melanin release with a dose effect: -86% and -45% statistically very highly significant (***), respectively with 0.1% and 0.05% of DE (dry extract, which is equivalent to 1 and 0.5 mg/mL).

In summary, in the present model of B16 murine melanocytes, melanin was dosed and reference molecules efficacy was well observed:

depigmentation with kojic acid (1 mM), known to inhibit synthesis of melanin, and pro-pigmentation with melanotan (100nM), which stimulates melanogenesis.

In the present evaluation, which represents a good model for melanogenesis by treatment of B16 melanocytes during 72h, HCR extract permitted to inhibit melanin synthesis. Thus, the HCR extracts were found to be effective in reducing melanin liberation and consequently being able to prevent sun-induced pigmentary spots, i.e. irregular and non-uniform pigmentation.

**Example 7 Evaluation of the antioxidant potential of *Hedychium coronarium* J. Koenig root extract (HCR extracts) in normal human keratinocytes (ROS detection with H$_2$DCFDA probe)**

1. Background Information

The aim of the present evaluation was to evaluate the antioxidant and antipollution effects of the extract HCR, against oxidative stress in normal human cultured keratinocytes, using the H$_2$DCFDA probe. Oxidative stress is induced by an environmental pollutant, benzo(a)pyrene (3,4-benzopyrene / BaP) or by a reference generator of reactive oxygen species (ROS), hydrogen peroxide (oxygenated water / $H_2O_2$).

Previously, a XTT assay was performed, to check the cells viability, in order to determine the concentrations to be tested, as described in Example 2.

BaP is produced by incomplete combustion of animal fat in contact with charcoal flames, in fumes of Diesel motors in case of insufficient combustion, in tar vapors. A tobacco cigarette produces 18 to 50 ng of BaP.

The dichlorodihydrofluorescein diacetate probe ($H_2DCFDA$/dichlorofluores *cin* diacetate) is a chemically reduced form of fluorescein used as an indicator for reactive oxygen species (ROS) in cells. It is cell-permeant and stable. When it penetrates the cell, acetate groups are cleaved by intracellular esterases. The resulting product is the dichlorodihydrofluorescein (or $H_2DCF$), non-fluorescent. In presence of ROS ($O^{2-}$, OH, NO, ONOO, ...) i.e. with oxidation, it is converted to the highly fluorescent product, dichlorofluorescein (or DCF). The probe oxidation is not specific of one type of ROS. The fluorescence is detected at wavelengths of 490nm (excitation) and 525nm (emission).

Biological Samples

Cells used in this study were normal human keratinocytes, extracted from abdominal plastic surgery (62 year-old woman Caucasian donor).

2. Evaluation of Antioxidant Effect ($H_2DCFDA$ Probe)

Assay Principle

Normal human keratinocytes were seeded in 96-well microplates and are then treated with the test compound to be tested for 24 hours, before adding the fluorescent probe for detection of ROS and stimulating or not by the pollutant or hydrogen peroxide.

This test is based on the use of a detection system, a probe, which is degraded and fluoresces on contact with ROS. This probe $H_2DCF$-DA is cleaved of two $CH_3$-COOH groups by cellular esterases; then the probe meets ROS present in the cell and looses two hydrogen atoms to become fluorescent DCF.

Cell Treatment and Melanin Dosage

Cells were seeded in 96-well microplates at 10 000 keratinocytes/well (30 000 cells/cm$^2$) in complete KSFM medium and were left to adhere for 24 hours at 37°C/5% $CO_2$.

Cells were treated with 3 non-cytotoxic concentrations of the extract to be tested (in sextuplicate, in non-supplemented medium) for 24 hours and incubated at 37°C/5% $CO_2$, in parallel to control conditions (untreated control or antioxidant positive reference).

An internal antioxidant positive reference was used in this study, used at 10 $\mu$M.

| | | *Concentrations to test* |
|---|---|---|
| **Without stimulation** | **Untreated control** | / |
| | **Positive control / reference molecule** | 10 µM |
| | **HCR extract** | 0.01 – 0.005 – 0.001 % of DE |
| | **Acetone solvent control** | 0.9% |
| **With $H_2O_2$ stimulation (1mM)** | **$H_2O_2$ control** | 1mM |
| | **Positive control + $H_2O_2$** | 10 µM + 1mM |
| **With BaP stimulation (36µM = 9 µg/mL)** | **BaP control** | 36 µM |
| | **Positive control + BaP** | 10 µM + 36 µM |
| | **HCR extract + BaP** | 0.01 – 0.005 – 0.001 % + 36 µM |

Probe Incorporation

After washing cells, the probe (Invitrogen D399, diluted at 50 $\mu$M in non-supplemented KSFM) was applied and the plates were incubated for 45 minutes at 37°C/5% $CO_2$.

Blanks wells WERE carried out without cells (but with the probe and tested products).

ROS Stimulation

After washing cells, BaP (Sigma CRM40071) or hydrogen peroxide solution (Sigma H1009) was applied in the expected wells (stimulated conditions, finally at $36\mu M$ and 1 mM respectively, in non-supplemented KSFM). For other wells (non-stimulated conditions), medium alone was applied. Plates were incubated for 20 minutes at 37°C/5% $CO_2$.

Expression of Results

After incubation, fluorescence was read at 490nm (excitation) and 525nm (emission) wavelengths (Tecan Safire II reader). Data are expressed as RFU (fluorescence units).
Data are collected and the averaged blank data is subtracted.

$$RFU_{\text{« sample »}} = RFU_{\text{raw data}} - RFU\,mean_{\text{« blank »}}$$

4. Results

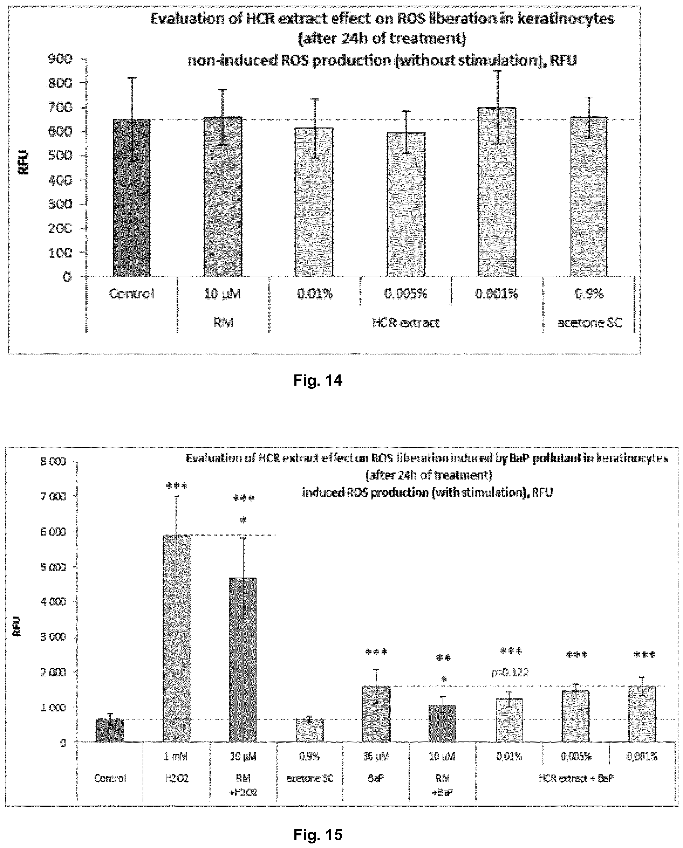

Results of antioxidant potential assay with or without ROS stimulation (by hydrogen peroxide or BaP), are presented in Figures 14 and 15. Therein, data are expressed in fluorescence units (RFU).
Significance (T-test) is indicated either comparatively to non-stimulated control (untreated of acetone solvent control: black stars), or comparatively to $H_2O_2$ or BaP -stimulated control (red stars).
The blank controls (without cells but with test compounds) have permitted to verify absence of interaction between the tested extract or positive reference and $H_2DCF$-DA probe.
Without ROS stimulation, there was no effect observed, in any of the different tests.
With hydrogen peroxide treatment, cells produce free radicals, as there is a stimulation of +808% of ROS production (comparatively to unstimulated control).
With BaP application, a stimulation is also observed, of +141% (comparatively to unstimulated acetone solvent control).
Concerning the reference molecule used, with ROS stimulation, an antioxidant effect is observed and is significant, as ROS production decreases by -21% (* with $H_2O_2$) and -33% (* with BaP).
The tested HCR extract showed efficiency at a concentration of 0.01%, as this highest tested concentration permits to inhibit ROS production, stimulated by the BaP pollutant (diminution of ROS detected): -23%, nearly significant (p=0.122).
In summary, the present evaluation showed that the tested HCR extract at the highest tested concentration (0.01%), shows antioxidant and antipollution potential.

**REFERENCES**

BURKILL H.M. (2000) - The Useful Plants of West Tropical Africa, Ed. Royal Botanic Gardens Kew, vol. 5, p.319.
DYMOCK W., WARDEN C.J.H., HOOPER D. (1892) - Pharmacographia Indica, Part V, p.417.
LODDIGES C. & Sons (1821) - The botanical cabinet, Ed. John & Arthur, n°507.
LOURTEIG A. (1972) - Le genre Hedychium à Madagascar (Zingiberacées) in Adansonia, ser. 2, 12 (1): p. 121-127.
NADKARNI K. M. (1976) - Indian material medica, Munbai : popular Praskan Private Limited, T.1, p. 608.
PERRIER DE LA BATHIE H. (1946) - Zingiberacees in HUMBERT H. - Flore de Madagascar, Imprimerie officielle (Tananarive), p. 4.
REES A. (1819) - The cyclopaedia ; or, Universal dictionary of Arts, Sciences, and Literature, Ed. Longman, Hurst, Rees, Orme & Brown (London), Vol. XVII, p. 522
RETZIUS, A. J., (1783) - Observationes botanicae, 3 : p. 73-74.
SHEEHAN T.J. (1958) - Florida State Horticultural Society - Zingiberaceae for Florida, 382-388.
SRAVANI T., PADMAA M.P. (2011) - Evaluation of anthelmintic activity of rhizomes of Hedychium spicatum Buch.Ham., Int. J. Res. Pharm. Sci., 2(1), 66-68.
SRAVANI T., PADMAA M.P. (2011) - Hedychium spicatum Buch.Ham. - An Overview - Pharmacologyonline, 2 : 633-642.
TURRILL W.B (1914) - Hedychium coronarium and Allied Species in Bulletin of Miscellaneous Information (Royal Gardens, Kew), Vol. 1914, n° 10, p. 368-372. www.efloras.org/ Flora of China, vol. 24, Zingiberaeae, Hedychium www.ipni.org/ The International Plant Names Index - Hedychium www.theplantlist.org/ The Plant List - Hedychium

**EP 3 342 465 A1**

www.tropicos.org/ Missouri Botanical Garden - Hedychium

## Claims

1. Use of *Hedychium coronarium J. Koenig* root or an extract thereof as a cosmetic agent in the protection against harmful environmental influences and/or in the protection against and/or the treatment of environmental damages deriving therefrom.

2. The use according to claim 1 for the protection against harmful environmental influences and/or the protection against and/or the treatment of environmental damages deriving therefrom by protecting or activating at least one of the cellular clean-up system.

3. The use according to claim 1 or 2 for the protection against harmful environmental influences and/or the protection against and/or the treatment of environmental damages deriving therefrom by protecting or activating at least one of the cellular clean-up system selected from the cellular metabolic system and/or the intracellular barrier system.

4. The use according to any one of the preceding claims for the protection against harmful environmental influences and the protection against and/or treatment of environmental damages deriving therefrom by protecting or activating at least one of the cellular clean-up system selected from

   - mitochondrial protection and/or activation,
   - lysosomal protection and/or activation, and
   - inhibition of cellular IL-8 release,
   - cellular production of β-endorphins,
   - inhibition of irregular melanine release, and
   - protection and/or activation of the cellular transmembrane system by claudin restoration.

5. The use according to any one of the preceding claims for the protection against harmful environmental influences and the protection against and/or treatment of environmental damages deriving therefrom by protecting or activating at least one of the cellular clean-up system selected from

   - mitochondrial protection and/or activation,
   - lysosomal protection and/or activation, and
   - protection and/or activation of the cellular transmembrane system by claudin restoration.

6. The use according to any one of the preceding claims for the protection against harmful environmental influences and the protection against and/or treatment of environmental damages deriving therefrom by protecting or activating at least one of the cellular clean-up system selected from

   - mitochondrial protection and/or activation, and
   - lysosomal protection and/or activation.

7. The use according to any one of the preceding claims for the protection against harmful environmental influences and the protection against and/or treatment of environmental damages deriving therefrom by protecting or activating at least one of the cellular metabolic system comprising

   - inhibition of cellular IL-8 release,
   - cellular production of β-endorphins and
   - inhibition of irregular melanine release.

8. The use according to any one of the preceding claims for the protection against harmful environmental influences and the protection against and/or treatment of environmental damages deriving therefrom by activation of the cellular antioxidant and radical oxygen species (ROS) defense system, such as in particular by reduction or inhibition of ROS liberation.

9. The use according to any one of the preceding claims, wherein environmental damages comprise UV-induced damages and toxin or pollutant induced damages.

**10.** A cosmetic composition comprising *Hedychium coronarium J. Koenig* root extract and optionally at least one additional agent selected from additional cosmetically active ingredients, solvents and auxiliaries.

**11.** The cosmetic composition according to claim 10 for the use as defined in any of the preceding claims.

**12.** A process for preparing a *Hedychium coronarium J. Koenig* root extract comprising the steps of

(a) providing a certain amount of pieces of the *Hedychium* plant to be extracted,
(b) extracting the *Hedychium* plant pieces with a suitable solvent or solvent mixture,
(c) filtration of the extraction mixture of step (b),
(d) concentration of the filtrate of step (c) by settling of the solid residues and decantation of the supernatant,
(e) filtration of the supernatant of step (d) to obtain the *Hedychium* extract as a clear solution
(f) adjustment of the clear solution of step (e) to the desired concentration using a suitable solvent to obtain the final extract in the desired concentration.

**13.** The process of claim 12, wherein in step (f) a mixture of water and glycerol is used for adjustment of the solution of the Hedychium extract.

**14.** The process of claim 12 or 13, wherein in step (f) a mixture of water and glycerol is used having a ratio 30 : 70.

**15.** The use according to any one of claims 1 to 9 or the cosmetic composition according to claim 10 or 11, wherein the concentration of the *Hedychium* extract is ≤ 10.0 % (weight / volume) based on the composition of the extract.

**FIGURES**

Fig. 1

Fig. 2

left column = without UVA irradiation
right column = with UVA irradiation

**Fig. 3**

**Fig. 4**

Fig. 5

Fig. 6

Fig. 7

Fig. 8

Fig. 9

**Fig. 10**

**Fig. 11**

EP 3 342 465 A1

Fig. 12

Fig. 13

Fig. 14

Fig. 15

Europäisches
Patentamt

European
Patent Office

Office européen
des brevets

# EUROPEAN SEARCH REPORT

Application Number

EP 16 20 7612

## DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| X | Anonymous: "Take Refuge from Irritated Skin", CoValence LABORATORIES , 8 July 2014 (2014-07-08), XP002769425, Retrieved from the Internet: URL:http://covalence.com/blog/take-refuge-irritated-skin [retrieved on 2017-04-11] * the whole document * ----- | 1-15 | INV. A61Q17/00 A61Q17/04 A61K8/97 A61Q19/02 A61Q19/08 |
| X | DATABASE GNPD [Online] MINTEL; February 2012 (2012-02), anonymous: "Sugar Scrub", XP002769426, Database accession no. 1726346 * the whole document * ----- | 1-15 | |
| X | DATABASE GNPD [Online] MINTEL; August 2009 (2009-08), anonymous: "Aliali Brightening Serum", XP002769427, Database accession no. 1157261 * the whole document * ----- | 1-15 | TECHNICAL FIELDS SEARCHED (IPC) A61Q A61K C07D |
| X | DATABASE GNPD [Online] MINTEL; June 2010 (2010-06), anonymous: "Daily Protecting Moisturizer SPF 15", XP002769428, Database accession no. 1388777 * the whole document * ----- | 1-15 | |

-/--

The present search report has been drawn up for all claims

| Place of search | Date of completion of the search | Examiner |
|---|---|---|
| Munich | 24 April 2017 | Ovens, Annabel |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons

& : member of the same patent family, corresponding document

EPO FORM 1503 03.82 (P04C01)

page 1 of 4

Europäisches
Patentamt

European
Patent Office

Office européen
des brevets

**EUROPEAN SEARCH REPORT**

Application Number

EP 16 20 7612

## DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| X | DATABASE GNPD [Online]<br>MINTEL;<br>July 2012 (2012-07),<br>anonymous: "Pores Refreshing Face Cleanser",<br>XP002769429,<br>Database accession no. 1826677<br>* the whole document * | 1-15 | |
| X | DATABASE GNPD [Online]<br>MINTEL;<br>February 2014 (2014-02),<br>anonymous: "Ultralight Grooming Spray",<br>XP002769430,<br>Database accession no. 2309026<br>* the whole document * | 1-15 | |
| X | DATABASE GNPD [Online]<br>MINTEL;<br>April 2010 (2010-04),<br>anonymous: "Conditioner",<br>XP002769431,<br>Database accession no. 1312549<br>* the whole document * | 1-15 | TECHNICAL FIELDS SEARCHED (IPC) |
| X | "Hedychium Coronarium Root Extract"<br>In: Joanne Nikitakis, Halyna P. Breslawec:<br>"International Cosmetic Ingredient Dioctionary and Handbook",<br>2014, Personal Care Products Council, Washington, D.C., XP002769432,<br>vol. 1, page 1461,<br>* the whole document * | 1-15 | |

-/--

The present search report has been drawn up for all claims

| Place of search | Date of completion of the search | Examiner |
|---|---|---|
| Munich | 24 April 2017 | Ovens, Annabel |

CATEGORY OF CITED DOCUMENTS

X : particularly relevant if taken alone
Y : particularly relevant if combined with another
document of the same category
A : technological background
O : non-written disclosure
P : intermediate document

T : theory or principle underlying the invention
E : earlier patent document, but published on, or after the filing date
D : document cited in the application
L : document cited for other reasons

& : member of the same patent family, corresponding document

EPO FORM 1503 03.82 (P04C01)

page 2 of 4

Europäisches
Patentamt

European
Patent Office

Office européen
des brevets

**EUROPEAN SEARCH REPORT**

Application Number

EP 16 20 7612

## DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| X | DATABASE WPI<br>Week 201406<br>Thomson Scientific, London, GB;<br>AN 2013-Q85083<br>XP002769433,<br>-& KR 2013 0106599 A (UNIV IND & ACADEMIC COOP IN CHUNGNAM NAT)<br>30 September 2013 (2013-09-30)<br>* abstract * | 1-15 | |
| X<br>A | CH 679 012 A5 (MRAK MAGDALENA)<br>13 December 1991 (1991-12-13)<br>* page 3, line 10 - page 3, line 15 *<br>* page 9, line 3 - page 9, line 8 *<br>* claim 1 * | 10,11,15<br>1-9,<br>12-14 | |
| X<br><br><br><br><br>A | DATABASE WPI<br>Week 201633<br>Thomson Scientific, London, GB;<br>AN 2016-030677<br>XP002769434,<br>-& CN 105 169 172 A (NANJING JINGYUN CHEM CO LTD) 23 December 2015 (2015-12-23)<br>* abstract *<br>* embodiment 1 * | 10,11,15<br><br><br><br><br>1-9,<br>12-14 | |
| X<br><br><br><br><br>A | DATABASE WPI<br>Week 201550<br>Thomson Scientific, London, GB;<br>AN 2015-391311<br>XP002769435,<br>-& CN 104 547 591 A (MENG X)<br>29 April 2015 (2015-04-29)<br>* abstract *<br>* ointment embodiment * | 10,11,15<br><br><br><br><br>1-9,<br>12-14 | |

TECHNICAL FIELDS
SEARCHED (IPC)

-/--

The present search report has been drawn up for all claims

| Place of search | Date of completion of the search | Examiner |
|---|---|---|
| Munich | 24 April 2017 | Ovens, Annabel |

CATEGORY OF CITED DOCUMENTS

X : particularly relevant if taken alone
Y : particularly relevant if combined with another
document of the same category
A : technological background
O : non-written disclosure
P : intermediate document

T : theory or principle underlying the invention
E : earlier patent document, but published on, or
after the filing date
D : document cited in the application
L : document cited for other reasons

& : member of the same patent family, corresponding
document

EPO FORM 1503 03.82 (P04C01)

page 3 of 4

Europäisches
Patentamt

European
Patent Office

Office européen
des brevets

# EUROPEAN SEARCH REPORT

## DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| X | DATABASE WPI<br>Week 201462<br>Thomson Scientific, London, GB;<br>AN 2014-M48930<br>XP002769436,<br>& CN 103 768 531 A (LIU Y)<br>7 May 2014 (2014-05-07) | 10,11,15 | |
| A | * abstract * | 1-9,<br>12-14 | |
| A,D | WO 02/056859 A2 (JOHNSON & JOHNSON CONSUMER [US]) 25 July 2002 (2002-07-25)<br>* the whole document * | 1-15 | |
| A | US 2004/086526 A1 (DANOUX LOUIS [FR] ET AL) 6 May 2004 (2004-05-06)<br>* the whole document * | 1-15 | |

| | | | TECHNICAL FIELDS SEARCHED (IPC) |
|---|---|---|---|

The present search report has been drawn up for all claims

| Place of search | Date of completion of the search | Examiner |
|---|---|---|
| Munich | 24 April 2017 | Ovens, Annabel |

EPO FORM 1503 03.82 (P04C01)

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons

& : member of the same patent family, corresponding document

## ANNEX TO THE EUROPEAN SEARCH REPORT
## ON EUROPEAN PATENT APPLICATION NO.

EP 16 20 7612

This annex lists the patent family members relating to the patent documents cited in the above-mentioned European search report.
The members are as contained in the European Patent Office EDP file on
The European Patent Office is in no way liable for these particulars which are merely given for the purpose of information.

24-04-2017

| Patent document cited in search report | | Publication date | Patent family member(s) | | Publication date |
|---|---|---|---|---|---|
| KR 20130106599 | A | 30-09-2013 | NONE | | |
| CH 679012 | A5 | 13-12-1991 | AU | 657283 B2 | 09-03-1995 |
| | | | CH | 679012 A5 | 13-12-1991 |
| | | | EP | 0514508 A1 | 25-11-1992 |
| | | | WO | 9209295 A1 | 11-06-1992 |
| CN 105169172 | A | 23-12-2015 | NONE | | |
| CN 104547591 | A | 29-04-2015 | NONE | | |
| CN 103768531 | A | 07-05-2014 | NONE | | |
| WO 02056859 | A2 | 25-07-2002 | AU | 2002251801 A1 | 30-07-2002 |
| | | | CA | 2435289 A1 | 25-07-2002 |
| | | | EP | 1363595 A2 | 26-11-2003 |
| | | | US | 2002155138 A1 | 24-10-2002 |
| | | | WO | 02056859 A2 | 25-07-2002 |
| US 2004086526 | A1 | 06-05-2004 | EP | 1382326 A1 | 21-01-2004 |
| | | | US | 2004086526 A1 | 06-05-2004 |

EPO FORM P0459

For more details about this annex : see Official Journal of the European Patent Office, No. 12/82

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description

- WO 2002056859 A2
- BR PI09055860 A2
- WO 2006053415 A1

### Non-patent literature cited in the description

- **JOHANN GERHARD KOENIG.** Observationes Botanicae. vol. 3, 73-74
- the Cyclopaedia; universal dictionnary Arts, Sciences and Littérature. **FRANCIS BUCHANAN-HAMILTON.** Abraham REES. vol. 17, 521-522
- Edible Medicinal and non medicinal Plants. *Flowers,* vol. 8, 853-860
- Medicinal plants used by women from Agnalazaha littoral forest (Sotuhestern Madagascar). *Journal of Ethnobiology and Ethnomedicine,* 2013
- **X. YAN et al.** *Traditional Chinese Medicines, Molecular Structures, Natural Sources, and Applications,* 1999
- **SHARMA et al.** *Phytochemistry,* 1975, vol. 14, 578
- Cosmetic Ingredient Handbook. The Cosmetic, Toiletry and Fragrance Association, Inc, 1988
- **BURKILL H.M.** The Useful Plants of West Tropical Africa. 2000, vol. 5, 319
- **DYMOCK W. ; WARDEN C.J.H. ; HOOPER D.** *Pharmacographia Indica,* 1892, 417
- The botanical cabinet
- **LOURTEIG A.** *Le genre Hedychium à Madagascar (Zingiberacées) in Adansonia,* 1972, vol. 12 (1), 121-127
- **NADKARNI K. M.** Indian material medica. Munbai : popular Praskan Private Limited, 1976, vol. 1, 608
- **PERRIER DE LA BATHIE H.** Zingiberacees in HUMBERT H. Flore de Madagascar, 1946, 4
- **REES A.** The cyclopaedia ; or, Universal dictionary of Arts, Sciences, and Literature. vol. XVII, 522
- **RETZIUS, A. J.** *Observationes botanicae,* vol. 3, 73-74
- **SHEEHAN T.J.** *Florida State Horticultural Society - Zingiberaceae for Florida,* 1958, 382-388
- **SRAVANI T. ; PADMAA M.P.** Evaluation of anthelmintic activity of rhizomes of Hedychium spicatum Buch.Ham. *Int. J. Res. Pharm. Sci.,* 2011, vol. 2 (1), 66-68
- **SRAVANI T. ; PADMAA M.P.** *Hedychium spicatum Buch.Ham. - An Overview - Pharmacologyonline,* 2011, vol. 2, 633-642
- **TURRILL W.B.** Hedychium coronarium and Allied Species in Bulletin of Miscellaneous Information (Royal Gardens, Kew). *Hedychium coronarium and Allied Species in Bulletin of Miscellaneous Information (Royal Gardens, Kew),* 1914, vol. 1914 (10), 368-372, www.efloras.org